# EUROPEAN PATENT APPLICATION

(11) **EP 4 819 158 A1**
(43) Date of publication of application: **07.10.2026**
(21) Application number: 26170369.8
(22) Date of filing: 02.04.2026
(51) Int. Cl.: G16H 50/20, G16H 50/70, G16H 20/40, G16H 50/30

(54) **METHODS AND SYSTEMS USING DEEP-LEARNING FOR IDENTIFYING PULMONARY VEIN ISOLATION NON-RESPONDERS**

(30) Priority: 04.04.2025 US 202519170306
(71) Applicant: Biosense Webster (Israel) Ltd., 2066717 Yokneam (IL)
(72) Inventor: ELIYAHU, Shiran, 2066717 Yokneam (IL); TSOREF, Liat, 2066717 Yokneam (IL); AMOS, Yariv, 2066717 Yokneam (IL)
(74) Representative: Carpmaels & Ransford LLP

(57) **Abstract**

A system trains a set of machine-learning models to predict which atrial fibrillation patients would respond to which treatments using ablation lines. The models identify an ablation line treatment for a patient based on atrial fibrillation-related features associated with the patient. The models estimate reconnection probabilities for ablation sites associated with at least one ablation line treatment. The models identify patient atrial fibrillation predictors associated with the patient, based on demographics and heart component dimension parameters associated with the patient, and use the at least one ablation line treatment, reconnection probabilities, patient atrial fibrillation predictors, and patient demographics to predict whether the patient would not respond to pulmonary vein isolation only treatment. In response to a prediction that the patient would not respond to pulmonary vein isolation only treatment, the system enables a healthcare provider to provide the at least one ablation line treatment for the patient.

## Description

### FIELD OF THE INVENTION

This disclosure relates to computer systems and computer implemented methods for the treatment of atrial fibrillation using pulmonary vein isolation.

### BACKGROUND

Atrial fibrillation (AF) is the most common diagnosed sustained arrhythmia and is characterized by rapid and irregular activation of the atria. Atrial fibrillation may be paroxysmal, lasting 7 days or less with or without intervention, or may be continuous beyond 7 days (persistent, PS-AF) or beyond 12 months (long-standing, LS-PS-AF). Permanent atrial fibrillation is the term used for longstanding persistent atrial fibrillation when any attempt to restore sinus rhythm has been abandoned or has proved impossible. *See, e.g.,* Kaba, R. A., Momin, A. & Camm, J. Persistent atrial fibrillation: The role of left atrial posterior wall isolation and ablation strategies. J. Clin. Med. 10, (2021).

Treatment strategies vary depending on the extent and duration of the atrial fibrillation. Pulmonary vein isolation (PVI) is a medical procedure that aims to restore normal heart rhythm and reduce or eliminate the symptoms associated with atrial fibrillation. As an example, paroxysmal atrial fibrillation is typically managed with wide antral circumferential ablation (WACA), i.e., providing radiofrequency energy in a site-by-site fashion along pre-defined lines of the atrium. However, a large proportion of patients with atrial fibrillation have PS-AF or LS-PS-AF, which are more difficult to treat, likely due to a wider spread of arrhythmogenic triggers and substrates outside of the pulmonary veins. In patients with advanced cases of atrial fibrillation, pulmonary vein isolation alone may not be sufficient, and long-term failure of a "pulmonary vein isolation only" is observed. The posterior wall of the left atrium is a common site for these changes and has become a target of ablation strategies to treat these more resistant forms of atrial fibrillation. Other common sites are the left and right carina and roof.

### BRIEF DESCRIPTION OF THE DRAWINGS

FIG. 1 illustrates a block diagram of an example system for using deep-learning for identifying pulmonary vein isolation non-responders, under an embodiment;
FIG. 2 illustrates a block diagram of inputs and outputs of the example system for using deep-learning for identifying pulmonary vein isolation non-responders, under an embodiment
FIG. 3A is a block diagram illustration of a deep learning system for identifying pulmonary vein isolation non-responders, under an embodiment;
FIG. 3B is a block diagram illustration of a deep learning structure, useful in the system of FIG. 3A, under an embodiment;
FIG. 4A-D are block diagram illustrations of an exemplary left atrium for deep learning system for identifying pulmonary vein isolation non-responders under an embodiment;
FIG. 5 illustrates a block diagram of wall dimension calculations, for using deep-learning for identifying pulmonary vein isolation non-responders, under an embodiment;
FIG. 6 illustrates a block diagram of a voltage map with ablation lines, for using deep-learning for identifying pulmonary vein isolation non-responders, under an embodiment;
FIG. 7 illustrates a block diagram of a vector velocity field, for using deep-learning for identifying pulmonary vein isolation non-responders, under an embodiment;
FIG. 8 illustrates a block diagram of bipolar window selections, for using deep-learning for identifying pulmonary vein isolation non-responders, under an embodiment;
FIG. 9 illustrates a block diagram of bipolar windows with different waves, for using deep-learning for identifying pulmonary vein isolation non-responders, under an embodiment;
FIG. 10 illustrates a block diagram of annotated types of beats, for using deep-learning for identifying pulmonary vein isolation non-responders, under an embodiment;
FIG. 11 is a flowchart that illustrates a computer-implemented method for using deep-learning for identifying pulmonary vein isolation non-responders, under an embodiment; and
FIG. 12 is a block diagram illustrating an example hardware device in which the subject matter may be implemented.

### DETAILED DESCRIPTION

In the following detailed description, numerous specific details are set forth in order to provide a thorough understanding of one example of the invention. However, it will be understood by those skilled in the art that the present invention may be practiced within the scope of the amended claims in myriad other examples or embodiments without conforming with the specific details of the particular illustrative example provided herein. In other instances, well-known methods, procedures, and components have not been described in detail so as not to obscure the present invention.

Embodiments herein enable using deep-learning for identifying pulmonary vein isolation non-responders, as described. A system trains a set of machine-learning models to predict which atrial fibrillation patients would respond to which treatments using ablation lines. The models identify an ablation line treatment for a patient based on atrial fibrillation-related features associated with the patient. The models estimate reconnection probabilities for ablation sites associated with at least one ablation line treatment. The models identify patient atrial fibrillation predictors associated with the patient, based on demographics and heart component dimension parameters associated with the patient, and use the at least one ablation line treatment, reconnection probabilities, patient atrial fibrillation predictors, and patient demographics to predict whether the patient would not respond to pulmonary vein isolation only treatment. In response to a prediction that the patient would not respond to pulmonary vein isolation only treatment, the system enables a healthcare provider to provide the at least one ablation line treatment for the patient.

For example, a system trains two graph convolutional neural networks and three dense layers in the neural networks to predict which atrial fibrillation patients would respond to which atrial fibrillation line treatments. A set of machine-learning models extracts maps from specific intracardiac-electrocardiogram data for a 61-year old patient named "Pat," and uses the maps 'data to identify a treatment which includes 6 ablation lines that include 33 ablation sites in the right atrium. Then the set of machine-learning models uses the ablation sites to estimate reconnection probabilities for the ablation sites. The set of machine-learning models receives demographics data and right atrial fibrillation parameters for Pat the patient, which are collectively used to predict that Pat's atrial fibrillation would become advanced. The set of machine-learning models uses the 6 ablation lines that include the 33 ablation sites in the right atrium, the highest estimated reconnection probabilities for the previous ablation sites, and Pat's atrial fibrillation predictors, which are collectively used to predict that Pat's atrial fibrillation will not respond to treatment using only pulmonary vein isolation only and predict that Pat's atrial fibrillation will respond to the 6 ablation lines treatment in Pat's right atrium. The system outputs these predictions to Pat's physician, which enables the changing of the treatment that Pat was going to receive.

The techniques of this disclosure relate to a system which uses machine-learning models that predict whether atrial fibrillation patients would not be responders to treatments using pulmonary vein isolation alone. This prediction is crucial for optimizing patient outcomes and tailoring treatment strategies. The system processes and trains on a multifaceted dataset that can include ground truth regarding patient outcome after X months (follow up), patient demographics, medical histories, medical imaging data such as specific intracardiac electrocardiogram (ECG) data, voltage maps/cases, and atrial cycle lengths +/- cycle length mappings for sites that meet catheter stability filters, ablation quality predictors, ablation reconnections, signal parameters and mapping analysis such as voltage, atrial dimensions and relationships between them, atrial cycle lengths, signal fractionations, and ablation reconnection probabilities. The approach involves several stages, including data preprocessing, deep learning model training, and post-processing of the models' proposals, recommendations, estimations, and predictions. As used herein the term "long-term success" is meant to include a period without atrial fibrillation as well as any arrhythmia ("atrial fibrillation and arrhythmia free period") following treatment, lasting between 3 to 24 months.

### Identification of Ablation Locations

The system **100** receives mapping data **102** for a heart, which include a left atrium 3-dimensional object and feature embeddings, which are generated using anatomical images produced by using a suitable medical imaging system or technique, such as an electrogram (EGM), body surface ECG, intracardiac ECG, and/or baseline recordings. The mapping data 102 includes both location information, of a catheter moving around the atrium, and electrocardiogram measurements, such as voltage at each location. The system **100** determines where to ablate by employing electro-anatomical maps **104** such as a segmentation map, PVC-based late potential map extracted during premature ventricular contraction beats, a late potential map, a bipolar voltage map, a conduction velocity map, a type of activation (fractionation, double potential, normal potential) map, a divergence map, and a curl map. The extraction of each map will be discussed in detail below in the sections describing vector velocity field divergence, conduction velocity, and type of activation map. The system **100** includes matrices **106** which may represent all different maps, such as an NxM sized feature matrix, where M=9 in this example since the system **100** typically has six different maps and the last three columns are defined by (x, y, z) positions of the vertices, and the corresponding adjacency matrix would have the size NxN.

For implementation, graph convolutional neural networks may be employed where the feature space is of the size NxM and is processed through a set of graph convolutional neurons. The system **100** can use softmax **108,** such as APPNP (Approximation of Personalized Propagation of Neural Predictions) softmax propagations which =1, 3, and 5, and ARMA softmax, such as with 21 channels, to generate the output layer. This architecture has been utilized successfully for deep learning processing of 3- dimensional objects. The output layer of the network is 6000x1 vector **110,** which represents the probability to ablate a given vertex in the patient's heart.

An example training set includes 60,000 cases (persistent and paroxysmal cases) from CartoNet with ablation data for each case. The target vector for the algorithm is a map of size 6000x1 with values of 0 or 1 for each vertex, where 1 represents a vertex that was ablated during the case due to extra targets. In order to obtain this map, pulmonary vein isolation ablation sites + Carina sites are filtered and the remaining ablation sites are projected to the anatomy, and any vertex with a distance of less than 4 mm (configurable) is marked as 1, while all other vertices are marked as 0. 80% of the cases are used for training, 10% of the cases are used for validation and 10% of the cases are used for testing the performance of the algorithm. Binary cross entropy loss function may be used to train this neural network.

The output of this neural network is further entered to a set of dense layers **112** which output ablation line predictions **114,** such as 13 types of ablation lines, and mean square error (MSE) loss may be used to predict the ablation lines, since more than one ablation line could be predicted by the neural network. The same set of 60,000 cases may be used to train this neural network, however the target vector for each case is a 13x1 vector, whereby 1 in this vector indicates that this ablation line was performed in the case. Using these techniques, the following ablation lines may be predicted:
left wide antral circumferential ablation (WACA),
right wide antral circumferential ablation,
left Carina,
right Carina,
roof line,
anterior line,
inferior line,
posterior line,
mitral line,
posterior wall debulking,
lateral wall debulking,
inferior wall debulking, and
other lines as desired.

A machine-learning system can be trained to identify large numbers of many different types of patients who have atrial fibrillation and whose ablation line procedure have higher probabilities of long-term success if the trainer of the machine-learning model has sufficient numbers of records of different types of patients whose ablation line procedures produced long term successes. If an insufficient number of records of different types of patients who experienced long term success with ablation line procedures are available, the training based on the decisions made by physicians that examined the atrial fibrillation patient and recommended on an ablation approach (and possibly also administer the treatment), rather than the actual observed outcome of the treatment, and thus overcome the technical setback resulting from insufficient training data. This alternative approach is based on the assumption that aligns with "Wisdom of the crowd" (WoC) or "hive brain" principle. According to this principle, in large scale (involving for example, hundreds of thousands or millions of unrelated cases or more), the majority of individuals make the correct decision. The use of physician recommendations in training the machine-learning model addresses the technical problem of data scarcity related to the actual outcomes of atrial fibrillation treatments, and leverages expert clinical judgment as a reliable surrogate for outcome data, thereby facilitating the generation of a robust and effective machine-learning model, despite the limited availability of outcome data.

### Contiguity Estimator

Pulmonary vein isolation may be performed with radio frequency energy in a site-by-site WACA, pattern (e.g., a WACA procedure) using an ablation catheter with an end point of the WACA procedure being a complete pulmonary vein isolation. After the last ablation of the WACA procedure, a physician can place a catheter at an ablation site to determine if spontaneous reconnection has occurred. If pulmonary vein reconnection has not occurred, a bolus of intravenous adenosine is administered to unmask any sites of dormant conduction. If required, an ablation may be performed at any site of reconnection to achieve pulmonary vein isolation again. In a study with 400 cases, those ablation sites where a physician had added "touch ups" were identified as contiguity sites, and a suitable network (e.g., system 100) was trained to estimate ablation sites in the original WACA procedure at the vicinity of those touch up sites as areas with potential reconnections sites. After a physician completes an ablation procedure (or after performing ablation of a specific ablation line), the system 100 then analyzes the real data from the ablation device-evaluating the probability of reconnections. By integrating data from demographics, electro-anatomical mapping, and the specific characteristics of the performed ablation sites, a model can update the predictions whether the patient will ultimately respond to the treatment. The data set may be split into training, validation, and test sets with a ratio of 80%, 10% and 10%. Binary cross entropy may be used as a loss function to train the neural network.

The contiguity inputs **116** can include positions (x,y,z) of ablation sites and the attributes of the ablation sites, such as impedance drop, radio frequency index, power, and force, and may be accessed by a sampler **118** used to generate contiguity matrices **120.** The contiguity matrices **120** may include a KxM contiguity feature matrix, where K represents the number of ablation sites in the case, and also include an adjacency matrix of KxK that represents the Euclidian distances between each of the ablation sites. The contiguity matrices **120** are input to a graph convolutional neural (GCN) network layer, which may be implemented by the contiguity softmax **122,** such as the APPNP and AMRA softmax, to produce the KX1 contiguity probability **124,** which store a 1 for each vertex in the heart which is predicted to need a reconnection investigation, and store a 0 for all other vertex in the heart. More detailed information about contiguity is available below in the section entitled "contiguity estimation."

Continuous values of demographic data **126,** such as age, body mass index (BMI), and left atria (LA) diameter serve as features for a dense layer network **128.** Categorical variables, such as gender, use of certain medical history, and the existence of hypertension, etc., may be converted to one hot encoding vector and serve as an input to the dense layer, in addition to the left atria predictors of atrial fibrillation: An AF features matrix **128** can include the predictors of atrial fibrillation, which is a set of 114 parameters that are calculated from 3-dimensional representations of the heart, cycle length and voltage maps. As the atrial fibrillation condition advances, the left atrium's form shifts from a tube-like shape to a spherical shape, and its volume expands. Evidence suggests a consistent link between the enlargement of the left atrium volume and the aggravation of both the severity and chronic nature of atrial fibrillation in affected patients. Therefore, factors such as atrial sphericity, low voltage areas, and cycle length across the seven anatomical structures of the left atrium (posterior wall, anterior wall, left lateral, right lateral, roof, mitral valve, and inferior walls) may assist in predicting the atrial fibrillation.

The system **100** includes a final dense layer **130** which receives inputs from the ablation line prediction **114,** the contiguity probability **124,** the demographic data **126,** and the AF features matrix **128,** and can also directly receive surface body surface ECG and intracardiac ECG, signals and parameters **132.** The final dense layer **130** can generate various outputs from these various inputs, depending on how the system **100** has been trained. F or example, if the system **100** has been trained only on pulmonary vein isolation only patients, the system **100** can identify a current patient's atrial fibrillation features that are similar to some of these historical pulmonary vein isolation only patients' atrial fibrillation features, and generate an output **134** that includes a prediction whether the existing patient who is a candidate for pulmonary vein isolation only, is likely to respond positively to pulmonary vein isolation only, or whether the patient will require a pulmonary vein isolation plus procedure instead. In another example, after the system **100** has been trained on all atrial fibrillation patient types with long-term outcomes, the output **136** may include a recommended ablation line treatment for the current patient with the specific atrial fibrillation features based on patients whose. atrial fibrillation features were similar and had a successful pulmonary vein isolation plus procedure. In yet another example, after the system **100** has been trained on all atrial fibrillation patient types with long-term outcomes, the output **138** may predict the probability of success regardless of the ablation approach.

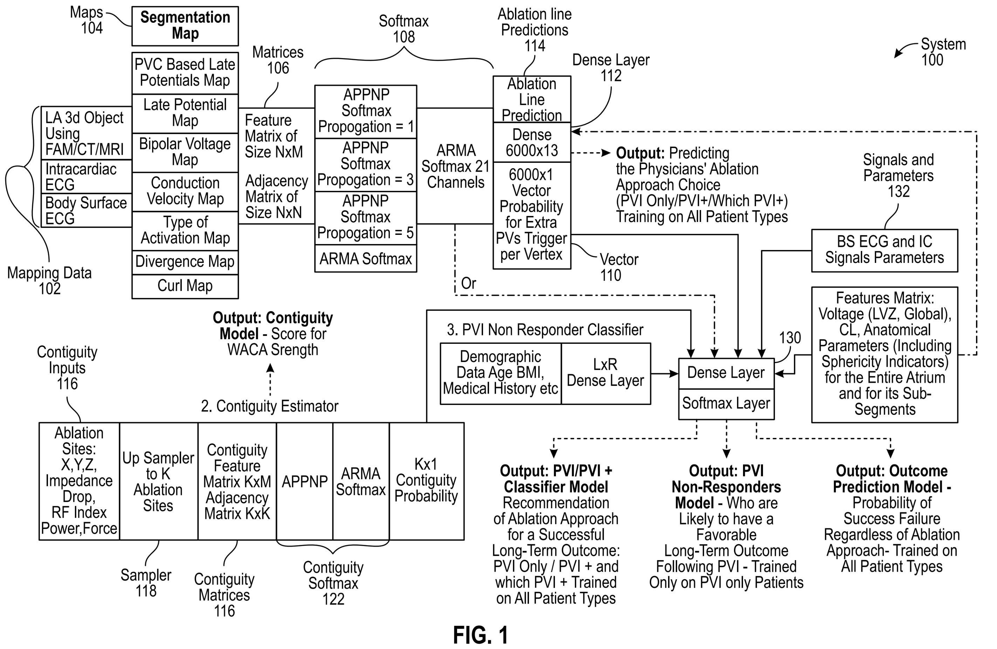

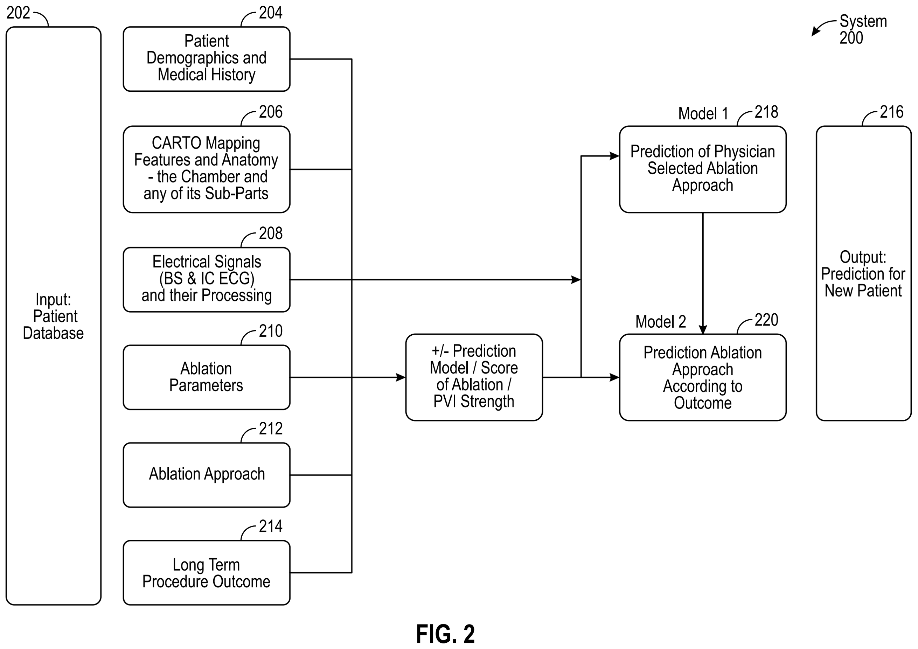

Fig. 2 depicts the inputs and outputs of a deep learning system **200** for identifying pulmonary vein isolation non-responders, which includes an input patient database **202** which provides data to **204 - 216** which generate outputs **216** - **220****.** The patient demographics and medical history **204** may be similar to the demographic data **126** in Fig. 1, CARTO^{®} mapping features **206** and anatomy for the chamber and any of its sub-parts may include voltage (LVZ, Global Voltage, and other calculations of voltage data, in addition to cycle length and different calculations of it, anatomical surface, volume, length, length, length, height, and width, and the ratios between them. all of which may be similar to mapping data **102** and maps **104** in in Fig. 1. Electrical signals **208** (Body Surface and Intra Cardiac Electrocardiogram) and their processing, may be similar to signals and parameters **132** in Fig. 1, Ablation parameters **210,** which may include baseline impedance, impedance drop, CF, power, time, ablation index, catheter stability, inter-lesion distance, temperature, etc., which may be similar to contiguity inputs **116** in Fig. 1, Ablation approach **212,** and long-term procedure outcome **214.** The output prediction for new patient **216** is based or the output prediction **218** of a physician-selected ablation approach for pulmonary vein isolation only or pulmonary vein isolation plus, which may be similar to the ablation line prediction **114** in Fig.1, and the output **220** for the prediction ablation approach may be for pulmonary vein isolation only or pulmonary vein isolation plus according to outcome, may also be similar to the ablation line prediction **114** in Fig.1 By adding the input for long-term procedure outcome **212,** the system **100** changes the output **214** for the prediction of a physician-selected ablation approach to a long-term procedure outcome **216.**

Deep learning systems may be utilized for planning ablation lines, such as wide antral circumferential ablation (WACA) lines and for checking how close the lines actually performed on the left atrium are to the planned ones. In addition, the problem of defining ablation lines is a segmentation problem. Thus, the input to such a system may be an anatomical map of the patient's heart and that the same type of deep learning structure may be utilized both for automatically segmenting the anatomical map as well as for defining the locations of the ablation lines.

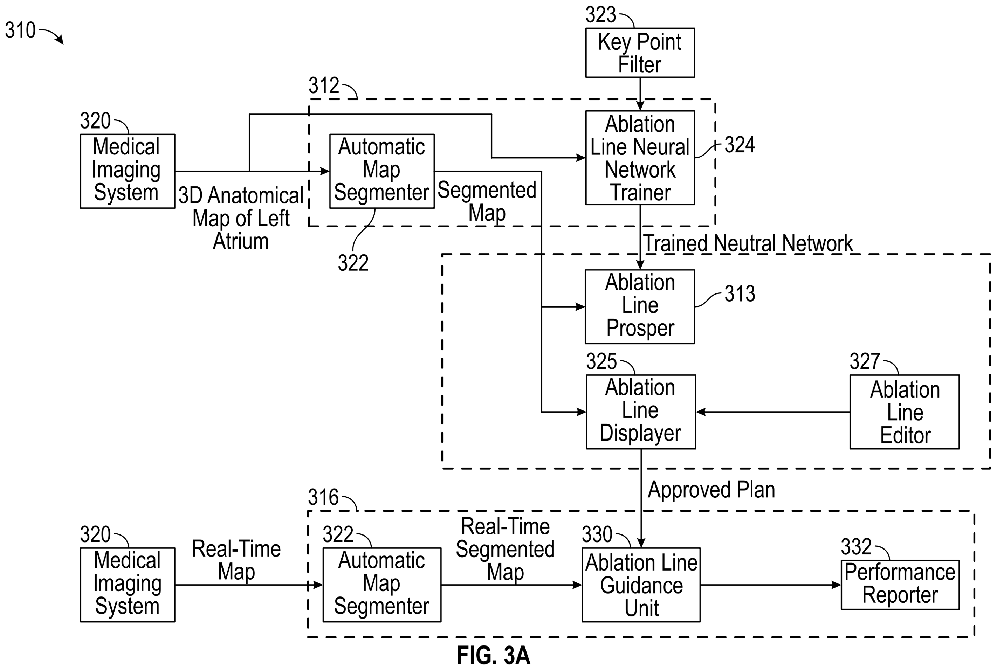

Reference is now made to FIG. **3A****,** which illustrates a deep learning system **310** for title. The deep learning system **310** comprises three sections, an ablation line trainer **312** which builds a trained deep learning unit for an ablation line proposer **313,** an ablation line recommender **314** which generates ablation line recommendations using the ablation line proposer **313,** and an ablation line guider **316** which provides ablation line guidance during a procedure. The ablation line trainer **312** may receive an anatomical map of the heart, which may be produced using an anatomical image produced by using a suitable medical imaging system **320,** or using a fast anatomical mapping (FAM) technique available in the CARTO^{®} system, produced by Biosense Webster Inc. (Irvine, Calif), or using any other suitable technique, or using any suitable combination of the above. The map/model of the heart onto which the recommended ablation line(s) may be rendered may comprise any suitable type of three-dimensional (3D) anatomical map produced using any suitable technique. The map may come from an intracardial electrocardiogram (ECG) and may comprise both location information, of a catheter moving around the atrium, and ECG measurements, such as voltage at each location.

The ablation line trainer **312** may comprise an automatic map segmenter **322** to segment the three-dimensional map of the heart received from the medical imaging system **320** into its various parts, such as posterior and anterior walls, roof wall, inferior wall, left lateral, septum, mitral, left atrial appendage (LAA) and 4 pulmonary veins (LSPV, LIPV, RSPV, RIPV), and an ablation line neural network trainer **324** to investigate thousands of ablation cases on different maps received from the medical imaging system **320,** along with their different ablation strategies, to generate a trained deep learning unit for the ablation line proposer **313.** The ablation line recommender **314,** which includes the ablation line proposer **313,** gives an initial recommendation for the typical ablation lines during a procedure, with the recommended line(s) being rendered on the anatomy, e.g., a model and/or anatomical map of the heart, segmented by the automatic map segmenter **322,** so that the physician may ablate as close as possible to the suggested line, thus yielding an ablation line that will be as close as possible to ablation lines preferred by key opinion leaders (KOLs) in the field. It is noted however that the ablation line proposer **313** may display the recommended ablation lines described herein with an ablation line displayer **325** on any digital depiction of the anatomy, whether an anatomical map or ultrasound imagery, including four-dimensional (4D) ultrasound imagery, or the like. Accordingly, as used herein, a "map" of anatomy refers to any such digital depiction onto which an ablation line may be projected.

The ablation line proposer **313** may allow the physician to specify the required ablation lines, which may be known ablation lines such as WACA, roofline, carina lines (right, left), posterior line, anterior line, inferior line, mitral line, posterior wall debulking, anterior wall debulking, inferior wall debulking, left lateral debulking, LAA isolation, etc. The ablation line recommender **314** may also comprise an ablation line editor **327** to enable the physician to review and modify the selected ablation line according to his or her requirements, preferences, and findings during the case. For example, a physician may want to edit the initial ablation lines such that it will contain triggers observed in the posterior wall.

The ablation line proposer **313** may segment WACA circles into sections and may recommend energy delivery per segment, i.e., the recommended ablation index (radiofrequency (RF) or irreversible electroporation (IRE)) and/or recommended distance between adjacent points, such as may be predefined for the particular ablation machine, such as the Carto^{®} machine, and/or may be configured by the physician prior to performing the ablation and/or may be per type of ablation line. The ablation line editor **327** may also permit the physician to update the recommended treatment delivery per segment. The ablation line recommender **314** may require the physician to approve the plan once he or she has reviewed all parameters.

The ablation line guider **316** provides ablation line guidance during a procedure. It may receive an anatomical map of the heart from the medical imaging system **320** during the procedure and may comprise the automatic map segmenter **322** to indicate the various elements of the heart during the procedure, an ablation line guidance unit **330,** and a performance reporter **332.** During the procedure, based on the catheter location, and the related ablation line from the approved plan, the ablation line guidance unit **330** may present a dynamic display of a next ablation site based on the previous site and on the recommended ablation line (approved or edited by the physician). The next ablation site may then be displayed based on a pre-defined distance e.g., 4 mm from the previous site, with a minimum distance from the suggested line and in the direction of advancement of the line.

The goal of an ablation procedure is to form effective lesions that can interrupt the abnormal electrical pathways causing arrhythmias. To this end, the ablation line guidance unit **330** may calculate an ablation index, indicating the quality of each radiofrequency (RF) ablation lesion during the procedure. The ablation index is a function of at least contact force, time, and power and thus, increases as an ablation operation continues. An instantaneous ablation index provides real-time feedback to the physician, enabling them to monitor and adjust their technique during the procedure.

Since the physician may define a "required ablation index" for a given ablation line and for a given segment within the line, the ablation line guidance unit **330** may present both the "required ablation index" and the instantaneous ablation index of the current site. This may enable the physician to yield the desired ablation index. The performance reporter **332** may provide a report to the physician based on his or her performance during the procedure with respect to the ablation plan produced by the ablation line recommender **314.** The report may include data such as the maximum and the median deviation from planned for each ablation line [in mm], the deviation from the planned location, including the number and the percentage of ablation points with a distance of more (or less) than the planned distance, and the deviation from the ablation planned for each ablation line. Note: this report may be particularly useful in training less experienced physicians.

The ablation line recommender **314** may be configured to always draw the recommended WACA (left and right) ablation lines. The same type of deep learning structure may be utilized and the same type of input provided to both the automatic map segmenter **322** and the ablation line neural network trainer **324.**

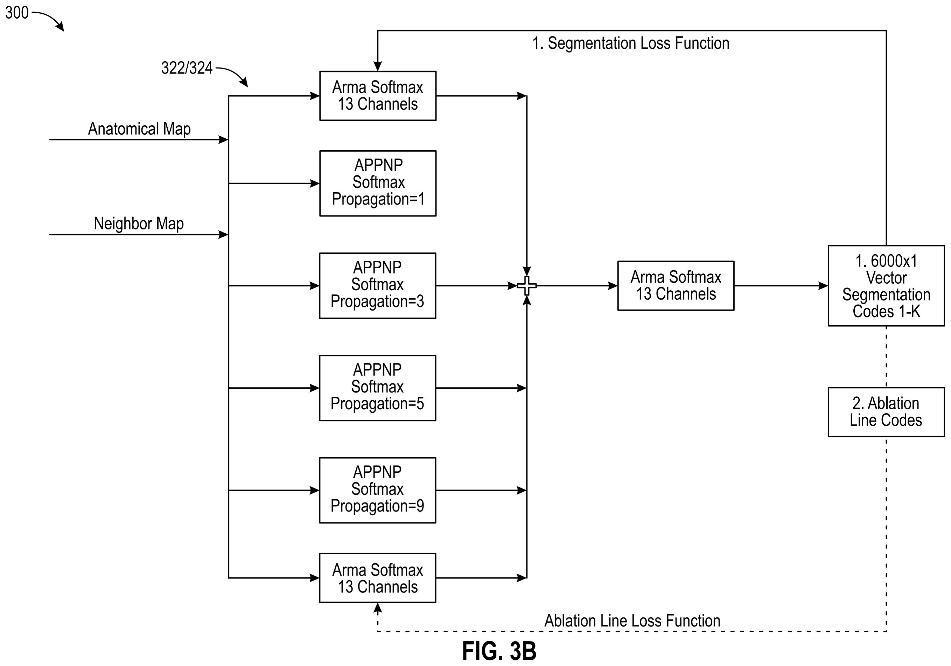

FIG. **3**B, to which reference is now made, shows the deep learning structure, discussed in more detail hereinbelow, and indicates that the input is the anatomical map, discussed above, and an adjacency map, discussed hereinbelow. FIG. **3B****,** however, shows two types of output, segmentation codes for the automatic map segmenter **322** and ablation line codes for the ablation line neural network trainer **324.** Segmentation codes may be a set of codes indicating the parts of the heart, or the parts of the atria, such as the 13 different parts of the atria. Ablation line codes may be a set of codes indicating a type of ablation line, as well as a code for all the areas which lack an ablation line. As discussed herein below, each training case may comprise an input map and an output map with each location labeled with one of the relevant codes, a segmentation code for training the automatic map segmenter **3*22*** or an ablation line code for training the ablation line neural network trainer **324.**

The initial data set may be composed of a sufficient number of paroxysmal and persistent atrial fibrillation cases (e.g., 5000 or more cases) with anatomical maps of each case and at least one ablation line that was performed in that case. The ablation lines are typically one of the following: (1) left WACA, (2) right WACA, (3) ostial PVI, (4) roof line, (5) right carina, (6) left carina, (7) posterior line, (8) inferior line, (9) mitral isthmus line, (10) anterior line, (11) Cavo tricuspid isthmus (CTI) line, (12) Superior Vena Isolation (SVCI), (13) LAA isolation. To prepare the initial data set to be used as input for training and testing the automatic map segmenter **322** and the ablation line neural network trainer **324,** an expert physician (such as a key opinion leader [KOL] in the field) initially may add manual annotations of ablation points on the anatomical maps of the cases, as generated by the medical imaging system **320.** Each point is associated with at least one of the ablation lines (1)-(13). Data preprocessing is performed whereby each ablation site is rendered on the anatomical map using a particular radius, such as a 5 mm radius.

In another step, each map be investigated, and maps with gaps/or blurred lines may be filtered out from training and testing sets. Following this, further data preprocessing may be performed in which the atrium is converted into a fixed dimension (e.g., by producing a constant mesh file with a fixed number of points, such as 2000 points and a 2000×2000 adjacency matrix, where all values are configurable, such as values of 1000, 2000, 4000, 10000 etc.). Feature extraction for 3-dimensional general segmentation may be used to build a deep learning framework for 3-dimensional segmentation, wherein graph data is characterized by improving the local features of each node. This may be achieved utilizing 1-triangle neighborhood spatial and structural features of each mesh face, formed of a point having a location (x, y, z) and a normal N to it, denoted by a vector (N_{X}, Ny, Nz). In a 1-triangle mesh, each face contains at most three adjacent faces. The 3-dimensional mesh structure data is characterized by M={V, E, F} of vertices, edges and face.

Therefore, the 1-triangle mesh is converted into a graph G={Features, Adjacency}. A face {F} on the mesh M has three corresponding nodes (vertices) on the graph {G}. The adjacency matrix {Adjacency} is a 6000×6000 matrix in our case. The adjacency matrix is based on {F} and represents the connections between vertices in M. In order to enhance the perception of the local area of the graph node, the spatial and structural geometric features are used based on a single vertex of the simplified mesh (in this example composed of 6000 vertices). The feature space is a 6000×9 matrix, for each vertex (a triangle composed of three vertices with 3 adjacent faces), the pre-processing utilizes the following features.
pᵢ-the normalized (xᵢ, yᵢ, zᵢ) position of vertex Vi in the Euclidian space, where i represents the three indices of the vertices;
spᵢ-spherical coordinate of vertex i (φᵢ, θᵢ, rᵢ), where φᵢ is normalized by π and θ is normalized by 2π; and
ni-normal of the vertex i defined by (xni, yni, zni).

The network architecture can include an automatic map segmenter **322** that may utilize a machine-learning model composed of two layers, the first layer is composed of six graph convolutional neural network units, and the second layer is a decision layer comprised of another graph convolutional neural (GCN) network with a Softmax unit. Each graph convolutional neural (GCN) network unit gives a prediction of a given vertex to be associated to each one of the target categories (anatomical structures or ablation line).

As shown in FIG. 3B, the anatomical map and the neighbor map of the adjacency matrix serve as inputs to all units in the network. Two types of graph convolutional neural (GCN) network units are used: 1) approximation personalized propagation of neural predictions (APPNP) and 2) auto regressive moving average layer (ARMA) that is highly efficient as a SoftMax layer for increasing the overall performance of the network during the training and cross validation stages and provides a more flexible frequency response, is more robust to noise, and better captures the global graph structure.

Personalized graph neural networks are described by Gasteiger, J., Bojchevski, A. & Unnemann, S. G.". Predict then propagate: Graph neural networks meet personalized PageRank. Int. Conf. Learn. Represent. (2018). Graph Neural networks with CNN ARMA filters have been described by Maria Bianchi, F., Grattarola, D., Livi, L. & Alippi, C. Graph Neural Networks with Convolutional ARMA Filters (2018).

The approximation personalized propagation of neural predictions (APPNP) unit models the relationship between graph convolutional neural (GCN) networks and the PageRank algorithm (a link analysis algorithm, originally designed by Google, which assigns a numerical weight to each node based on the quantity and quality of links point to it) and extends it to a personalized PageRank. It uses the information from a large, adjustable neighborhood for classifying each node. The model is computationally efficient and outperforms several state-of-the-art methods for semi-supervised classification on multiple graphs. The approximation personalized propagation of neural predictions (APPNP) has 13 channels with a multi-layer perceptron (MLP) having two hidden layers with 128, and 64 neurons in each layer, respectively.

The network weights are trained using a weighted category cross entropy loss function. The automatic map segmenter **322** may provide the segmentation codes to its segmentation loss function while the ablation line neural network trainer **324** may provide the ablation line codes to its ablation line loss function. In an exemplary embodiment, the training set contains 5000 maps. Each map is represented using three matrixes, a 6000×9 feature matrix, a 6000×6000 adjacency matrix and a 6000×1 target matrices, one for the segmentation codes and one for the ablation codes. For the ablation codes, zero represents no line while non-zero represents the ablation line ID.

Once the neural network of the automatic map segmenter **322** is trained, the automatic map segmenter **322** may segment the anatomical map of each atrium and may provide the segmented map to the ablation line displayer **325.** From segmentation to line, the ablation line proposer **313** may create a centralized line with potential locations for point-by-point ablations on the anatomical map._Point-by-point ablation "dots" may be updated on the fly based on actual ablation performed by the physician during each case. The mechanism for updating the dots may involve minimizing the distance between the planned line and the next dot in the direction of advancement of the ablation line.

As discussed, the system **310** may use mapping data and automatic segmentation data to recommend which of the ablation lines is more suitable for a given case. The recommendation may be set based on indications from a specific research hospital or specific physicians (e.g., a key opinion leader [KOL] in the field of cardiac ablation) or based on data from a multitude of physicians and/or research hospitals. In an alternative embodiment, the structure used for both the automatic map segmenter **322** and the ablation line trainer **324** may comprise a classifier operating on data of the intracardiac electrocardiogram (ECG). During intracardiac ECG recordings, catheters are inserted into the heart to measure the electrical activity of the heart. At each anatomical location, the electrical signals are represented as voltage amplitude.

In the alternative embodiment, the classifier may be any suitable classifier, such as a Random Forest Classifier, a Support-Vector Machine (SVM) classifier or a deep learning classifier. Each case in the training dataset may be preprocessed with an indication of whether one of given number of ablation lines (e.g., 14) were performed by a key opinion leader (KOL), so the target is a matrix of size N×14 where N represents the number of cases in the training dataset.

Input Data: electrocardiogram (ECG) data from the intracardiac ECG is saved as an additional feature matrix of 6000×22. The following triggers and substrate maps from the intracardiac ECG data are used as feature spaces:
1) "Finder" Focal source per vertex (6000×1) represents a number of S-waves per second;
2) "Finder" RAP-each vertex is represented by a number of rotational patterns per second;
3) CL-Cycle Length mapping-each vertex is represented by a cycle length in msec;
4) STD CL- Cycle length standard deviation-each vertex is represented by a cycle length standard deviation per second;
5) Spatial temporal dispersion-each vertex represents a percentage of spatial temporal dispersion (out of 10-30 seconds of the recording);
6) Periodic Spatial temporal dispersion-each vertex represents a percentage of periodic spatial temporal dispersion (out of 10-30 seconds of the recording);
7) Voltage Map-each vertex represents a bipolar amplitude in mV;
8) CLM ROI-each vertex represents an existence of cycle length region of interest, based on a cutoff of cycle length<minimum CL+15 msec and STD CL<15 msec;
9) Segmentation hot one encoding is the output of the automatic map segmenter **322:** a matrix of size (6000×K) where K represents the number of anatomical structures (13 for the LA), (output from segmentation algorithm). Ablation line encoding is the output of the ablation line trainer **324;**
10) Global voltage per segmentation region (6000×1) mV; and
11) Low voltage zone areas are regions within the left atria where the recorded voltage amplitudes are relatively low compared to surrounding areas. For example, these may be areas whose voltages are between 0.2-0.5 mV. Typically, a scar may be defined as an area whose voltage is below 0.2 mV. It will be appreciated that these thresholds may differ based on operator decision in the procedure.

The classifier may receive a feature matrix as input, which may have dimensions of 6000×M, where 6000 represents the number of vertices on the map, and M represents the number of features (focal, substrate related, ripple, frequency, anatomical location, etc.). The target vector has dimensions of K×1, where K indicates the number of ablation approaches or IDs. For this embodiment, the data may be from successful cases of the procedure, specifically those with a 12-month freedom from any arrhythmia. For each of these cases, the K×1 vector will be set to 1 if a particular ablation approach was performed during the procedure.

The neural networks of the classifier may utilize a set of dense nets, where the last layer will consist of a SoftMax layer with K neurons in the output layer. This modification enables the prediction of the K ablation approaches. It will be appreciated that the systems and methods described herein may be in various local or distributed computers (e.g., a cloud-based processing systems) including but not limited to the form described by U.S. Patent No. 11,198,004 or U.S. Patent Publication No. 2020/0352652A1, owned by Applicant and incorporated herein by reference. Actual ablation line data may be noisy and the ablation lines they indicate may not be the ideal lines. In another embodiment, the system **310** may additionally comprise a key point filterer **323** to review the noisy ablation line data and, based on the segmentation map received from the automatic map segmenter **322,** may find those ablation points in the ablation line data which may be defined as key points (i.e., the important points along the ablation line which should not have been missed) as defined by an expert, such as a key opinion leader (KOL), associated with it.

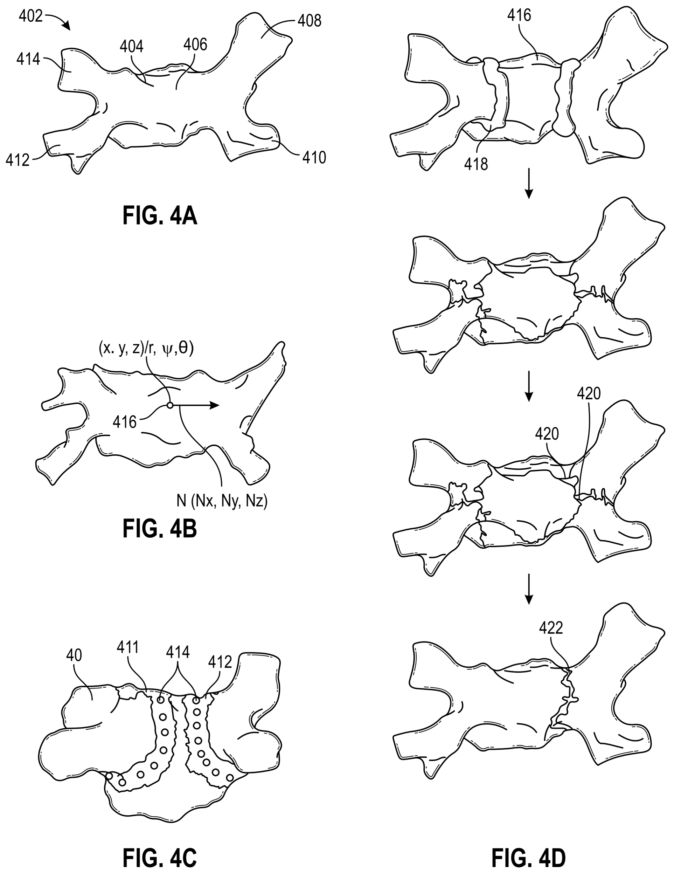

Reference is now made to FIG. 4A, which illustrates an exemplary left atrium 4**02** in the posterior anterior (PA) view, which illustrates multiple exemplary areas, such as a roof wall 404, and a posterior wall 406. To its right, is a right superior pulmonary vein (RSPV) 408 and a right inferior PV (RIPV) 410, and to its left is a left inferior PV (LIPV) 412 and a left superior PV (LSPV) 414. To build a deep learning framework for 3-dimensional segmentation, graph data is characterized by improving the local features of each node. such as by utilizing 1-triangle neighborhood spatial and structural features of each mesh face, as depicted in FIG. 4B, which shows a mesh face formed of a point 4**16,** having a location (x, y, z) and a normal N to it, denoted by a vector (N_{X}, Ny, Nz). FIG. 4C depicts an example of ablation lines 4**18** and **420** with potential locations for point-by-point ablations on the anatomical map, such as the point-by-point ablations indicated by dots 4**22.**

Actual ablation line data may be noisy and the ablation lines they indicate may not be the ideal lines. In another embodiment, shown in FIG. 3A to which reference is now made, the system 3**10** may additionally comprise a key point filterer 3**23** to review the noisy ablation line data and, based on the segmentation map received from the automatic map segmenter 3**22,** may find those ablation points in the ablation line data which may be defined as key points (i.e., the important points along the ablation line which should not have been missed) as defined by an expert, such as a key opinion leader (KOL), associated with it. Typically, these key points may be points at the intersection of two parts of the atrium.

This is shown in FIG.4D, to which reference is now made. The input 4**16** to key point filterer 3**23** may be an image or a mesh of the left atrium with the actual ablation lines, which may have been segmented 4**18** by the automatic map segmenter 3**22.** The key point filterer 3**23** may detect the key points 4**20** by comparing the ablation points of each case to the intersection points on the segmented map for that case and selecting those ablation points closest to the intersection points. The key point filterer 3**23** may combine key points from multiple cases to generate resultant ablation lines 4**22** for training which may be thinner and better defined than otherwise. The key point filterer 3**23** may provide the resultant ablation lines 4**22** to the ablation line neural network trainer 3**24** for training.

### Contiguity Estimation

Pulmonary vein isolation may be performed with radio frequency energy in a site-by-site WACA, pattern (e.g., a WACA procedure) using an ablation catheter with an end point of the WACA procedure being a complete pulmonary vein isolation. After the last ablation of the WACA procedure, the physician places a catheter at an ablation site to determine if spontaneous reconnection has occurred. If pulmonary vein reconnection has not occurred, a bolus of intravenous adenosine is administered to unmask any sites of dormant conduction, if required, an ablation may be performed at any site of reconnection to achieve pulmonary vein isolation again. In a study with 400 cases, those sites where the physician had added "touch ups" as sites of contiguity were identified and a suitable network (e.g., FIG. 1) was trained to estimate ablation sites in the original WACA procedure at the vicinity of those touch up sites as areas with potential reconnections sites. The data set may be split into training, validation, and test sets with a ratio of 80%,10% and 10%. Binary cross entropy may be used as a loss function to train the neural network.

The position of ablation sites and the attributes of the ablation sites may be used to generate a KxM feature matrix, where K represents the number of ablation sites in the case. An adjacency matrix of KxK that represents the Euclidian distances between each of the ablation sites may also be calculated as part of the input to the graph convolutional neural (GCN) network layer.

The following are examples of features that may be calculated for this task:
]normalized x: (x-minimum(x))/(maximum(x) - minimum(x))
normalized y: (y- minimum(y))/(maximum(y) - minimum(y))
normalized z: (z- minimum(z))/(maximum(z) - minimum(z))
normalized x by maximum range: (x- minimum(x))/(maximum range)
normalized y by maximum range: (y- minimum(y))/(maximum range)
normalized z by maximum range: (z- minimum(z))/(maximum range)

The following features are "circle based" features and are used to establish orientation-positioning, inferior-roof and anterior-posterior:
arctan(x/y)
arctan(y/z)
arctan(x/z)

The next set of features are specifically designed to add specific information in both left - right WACAs, posterior-anterior, and roof-inferior. Each ablation site may be normalized based on its association with a WACA ring:
mean x ring - mean of x-values of a ring
mean y ring - mean of y-values of a ring
mean z ring - mean of z-values of a ring
normalized x ring: (x ring-minimum(x ring))/(maximum(x ring)-minimum(x ring))
normalized y ring: (y ring-minimum(y ring))/(maximum(y ring)-minimum(y ring))
normalized z ring: (z ring-minimum(z ring))/(maximum(z ring) minimum(z ring))
normalized x ring by maximum range: (x ring - minimum(x ring))/( maximum range)
normalized y ring by maximum range: (y ring - minimum(y ring))/( maximum range)
normalized z ring by maximum range: (z ring - minimum(z ring))/( maximum range)
arctan(mean x ring / mean y ring)
arctan(mean y ring / mean z ring)
arctan(mean x ring / mean z ring)
ring XY - (range ring x) * (range ring y)
ring XZ - (range ring x) * (range ring z)
ring YZ - (range ring y) * (range ring z)
ring magnitude: [number of ablation sites in the ring] / [number of ablations sites]

The WACA random forest algorithm may be trained on a set of 25 position features and additional 8 ablation site characteristics taken from CARTO^{®} VisiTag Export:
duration time (total duration of ablation site in seconds)
average force
maximum temperature [°C]
maximum power [W]
base impedance[Ω]
impedance drop [Ω]
force time integral (FTI)
ablation index

Sphere stability maximum may be calculated as the maximum Euclidian distance between the center of an ablation site to all ablation sites associated with the ablation site. The unit of this feature is [mm].

Average sphere stability velocity may be calculated as the average Euclidian distance between the center of an ablation site to all ablation sites associated with the ablation site, divided by the duration of ablation in seconds. The units of this feature is [mm/sec].

Line stability maximum - minimizing least square minimization for the distance of a set of sites to a line in 3-dimenaional space. Line stability maximum may be calculated as the maximum distance between ablation sites and the estimated least square line. The unit of this feature is [mm].

Average line stability velocity may be the average distance between ablation sites and the estimated least square line divided by the duration of the ablation site. The unit of this feature is [mm/sec].

Maximum valid impedance drop may be related to a set of ablation sites that are less than 1mm from an ablation line.

Maximum radio frequency index - maximum valid ablation index may be related to a set of ablation sites with less than 1mm from an ablation line.

Number of ablation disconnections may be the number of times an ablation index and an impedance drop get disconnected (from tissue, with values of -10000).

Minimum distance post may be the minimum Euclidian distance between the center of an ablation site to one of the ablation sites forming the next ablation site.

Minimum distance previous may be the minimum Euclidian distance between the center of an ablation site to one of the ablation sites forming the previous ablation site.

Number of ablation sites forming an ablation site.

Number of valid ablations in line may be estimated based on a least square model. A valid site is assumed if its distance to an ablation line is less than 1 mm.

The next set of features measure the density of ablation sites associated with a given ablation site:
Number of ablation sites in 3 mm radius from the current ablation site
Number of ablation sites in 4 mm radius from the current ablation site
Number of ablation sites in 5 mm radius from the current ablation site
Number of ablation sites in 6 mm radius from the current ablation site
Number of ablation sites in 7 mm radius from the current ablation site
Number of ablation sites in 8 mm radius from the current ablation site
Number of ablation sites in 9 mm radius from the current ablation site
Number of ablation sites in 10 mm radius from the current ablation site
Number of ablation sites in 11 mm radius from the current ablation site
The time difference from the next ablation site
The time difference from the previous ablation site

### Demographics and left atrium predictors of atrial fibrillation

Continuous values of demographic parameters such as age, body mass index (BMI), and left atria (LA) diameter serve as features for a dense layer network. Categorical variables, such as gender, use of certain medical history, and the existence of hypertension, etc., may be converted to one hot encoding vector and serve as an input to the dense layer, in addition to the left atria predictors of atrial fibrillation:

The left atria predictors of atrial fibrillation is a set of 114 parameters that are calculated from 3- dimensional representations of the left atria, cycle length and voltage maps. As the atrial fibrillation condition advances, the left atrium's form shifts from a tube-like shape to a spherical shape, and its volume expands. Evidence suggests a consistent link between the enlargement of the left atrium volume and the aggravation of both the severity and chronic nature of atrial fibrillation in affected patients. Therefore, factors such as atrial sphericity, low voltage areas, and cycle length across the seven anatomical structures of the left atrium (posterior wall, anterior wall, left lateral, right lateral, roof, mitral valve, and inferior walls) may assist in predicting the atrial fibrillation.

The left atria predictors of atrial fibrillation report displays 114 parameters categorized into groups, as follows:
16 parameters in the atrium size parameters group
   7 parameters in the atrium sphericity parameters group
24 parameters in the low voltage zones parameters group
36 parameters in the global voltage parameters group
16 parameters in the cycle length parameters group
114 total parameters in the parameters groups

### Atria Size Parameters

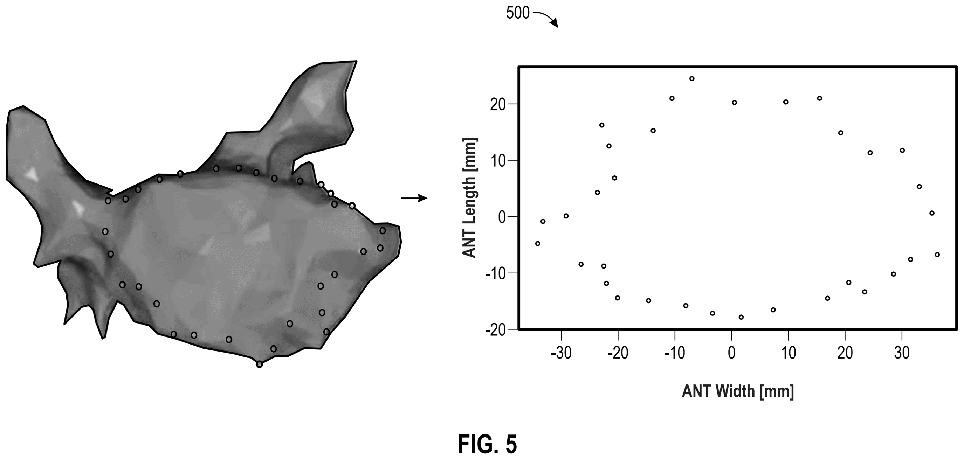

An automated segmentation algorithm such as disclosed in commonly-assigned U.S. Patent Publication No. 2023/0397950, filed June 9, 2023, and which is hereby incorporated herein in its entirety, may be used to measure the width and the height for seven anatomical structures of the left atria (posterior wall, inferior wall, anterior wall, roof wall, septum wall, left lateral wall, mitral valve) as well as the total volume and surface of the left atria. The width (or height) of an anatomical structure is mathematically defined as the distance in millimeters between the maximum and minimum points along the x-axis (or y-axis) within the 2-dimensional plane. A schematic example is depicted in FIG. 5, showing the calculation of anterior wall dimensions using atria sphericity parameters such that, based on the atria size measurements, the ratio of width to height of each of the seven structures of the left atria are displayed. This approach presupposes a consistent characteristic across each of the anatomical structures, implying that one dimension of the left atria consistently exceeds the other. For instance, it is anticipated that the anterior is consistently elongated anteriorly relative to its width.

Low voltage zone parameters may also be calculated as a voltage map based on pre-ablation electro-anatomical points that may be generated in the background, identifying three distinct low voltage zones:
scar tissue with voltage below 0.2mV
border-zone voltage regions with voltage ranging from 0.2 to 0.5mV
regions with voltage under 0.5mV.

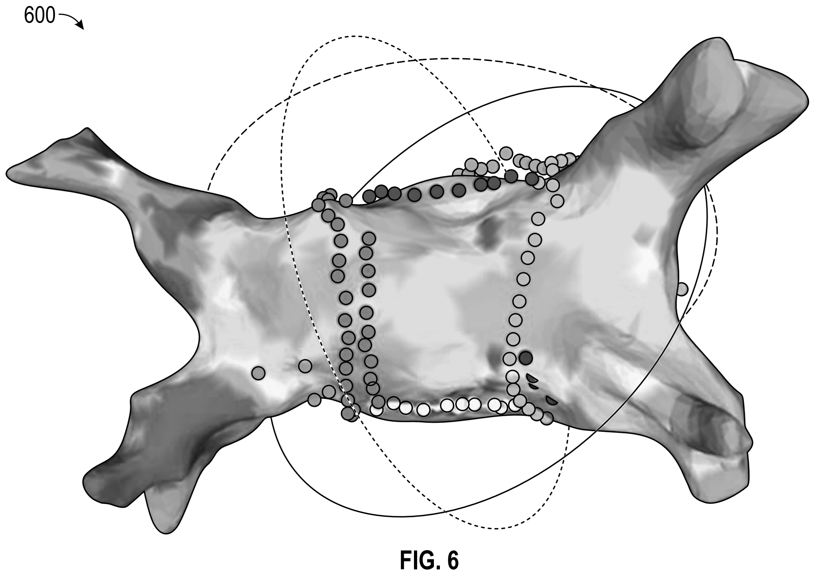

An automated segmentation algorithm quantifies these low voltage zone regions, expressed as their percentage of eight specific sub-anatomical structures: posterior wall, inferior wall, anterior wall, roof wall, septum wall, left lateral wall, mitral wall, left atrial appendage, and the left atria excluding the four pulmonary veins and the appendage. Consequently, a total of 24 parameters may be derived, resulting from the computation of 3 low voltage zone (LVZ) regions as a percentage of each of the aforesaid eight anatomical regions. An example of the background voltage map with ablation lines that may be used to calculate the low voltage zone parameters is depicted in FIG. 6. As shown, the red and orange regions reflect low voltage of below 0.2 mV; the yellow and green regions reflect low voltage of between 0.2 - 0.5 mv; the light blue regions reflect voltage above 0.5 mV, the dark orange spheres reflect ablations on the left WACA line, the light orange spheres reflect ablations on the right WACA, the purple spheres reflect ablations on the roof line and the yellow spheres reflect ablations on the inferior line.

### Global Voltage Parameters

An automated algorithm calculates four voltage parameters across the eight sub-anatomical structures (posterior wall, inferior wall, anterior wall, roof wall, septum wall, left lateral wall, mitral wall, left atrial appendage, and the left atria excluding the four pulmonary veins and the appendage). The four voltage parameters are the 25th, 50th, median and 75th voltage percentiles, as well as the range between the 25th percentile and the 75th percentile.

### Cycle Length Parameters

An automated algorithm calculates four global cycle length parameters across the eight sub-anatomical structures (posterior wall, inferior wall, anterior wall, roof wall, septum wall, left lateral wall, mitral wall, left atrial appendage, and the left atria excluding the four pulmonary veins and the appendage). The four cycle-length parameters are the 25th, 50th, median and 75th cycle length percentiles, as well as the range between the 25th percentile and the 75th percentile.

In one aspect, a pulmonary vein isolation non responders network may be trained using the features noted above. A collection of 1,300 cases, encompassing both persistent and paroxysmal conditions treated with only the pulmonary vein isolation procedure, may be utilized to train the deep-learning algorithm. The dataset may be divided into an 80% training set and a 20% testing set. Each case may be labeled as either a responder or a non-responder to pulmonary vein isolation. The dense layer depicted in the lower portion of FIG. 1, may be trained using the binary cross-entropy loss function.

### Vector Velocity Field Divergence and Conduction Velocity:

A vector velocity field may be defined for local activation time. Detection of local activation time and normalization of local activation surface may be modeled as: $T \left(x, y\right) = {ax}^{2} + {by}^{2} + cxy + dx + ey + f$ where (x,y) represents the surface of the catheter in two-dimensional space after projecting 3-dimensional space into 2-dimensional space, assuming that the catheter is placed on a 2-dimensional surface. This assumption is validated by checking that the two higher eigen values preserve 95% of the energy.

### Vector velocity field Divergence and Conduction velocity.

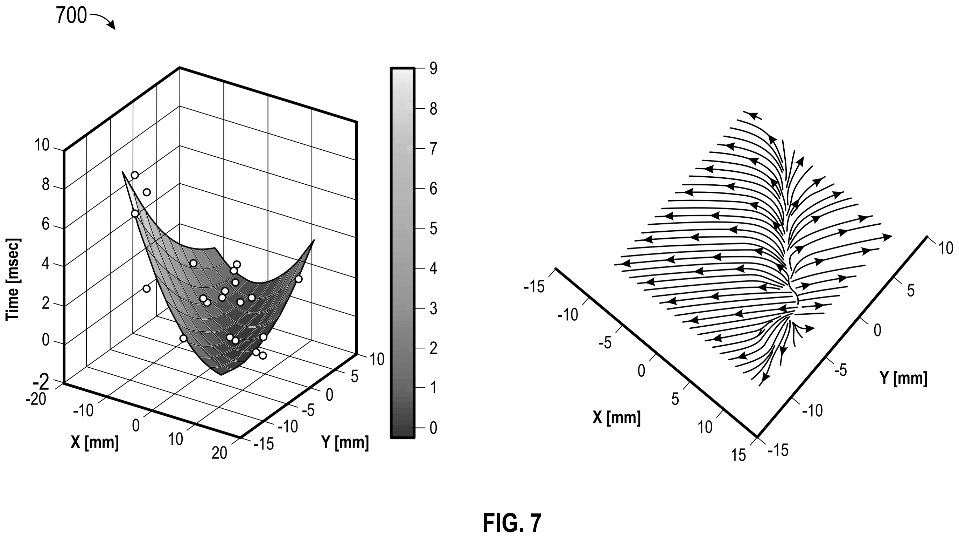

Correspondingly, a vector velocity field as schematically depicted in FIG. 7 may be estimated as: ${V}_{e} = \left[\begin{matrix}\frac{Tx}{{T}_{x}^{2} + {T}_{y}^{2}} \\ \frac{{T}_{y}}{{T}_{x}^{2} + {T}_{y}^{2}}\end{matrix}\right] , {T}_{x} = \frac{\partial T \left(x, y\right)}{\partial x} , {T}_{y} = \frac{\partial T \left(x, y\right)}{\partial y}$

In this depiction, the surface is fitted to local activation time, with the XY surface representing the location of the electrodes in 2-dimensional space and the Z axis representing the time difference of the local activation from a reference point to a normal QRS time. Such a vector velocity field may provide the raw data for obtaining curl, divergence and conduction velocity maps. The conduction velocity is measured as an average of conduction velocities in a region of 2 mm radius of the velocity vectors field. Curl and divergence are mathematical operators that may be applied to a velocity vector field as described in C, Dallet et al. Cardiac Propagation Pattern Mapping With Vector Field for Helping Tachyarrhythmias Diagnosis With Clinical Tridimensional Electro-Anatomical Mapping Tools, IEEE Trans Biomed Eng. 2019 Feb. 66(2): 373-382. Curl offers a gauge of the curvature in the propagation of the wavefront by calculating the circulation of the vector-valued velocity. On the other hand, the divergence field is a mathematical tool that assesses the propensity of the conduction velocity (CV) vectors around each point to either converge or diverge from it. Specifically, if the divergence value is negative, it indicates that the conduction velocity is moving inwards towards the point, creating a "sink" in the conduction velocity field where wave-fronts clash. Conversely, if the divergence value is positive, it suggests that the conduction velocity is moving outwards from the point, forming a "source" in the conductivity field, the point from which the wave-front expands.

### Type of Activation Map

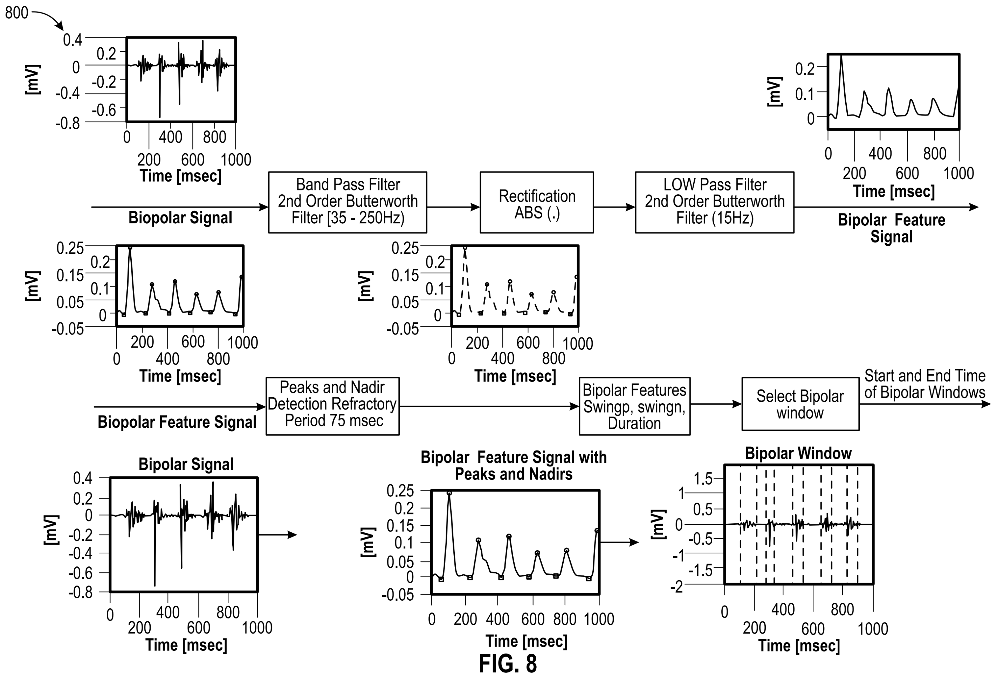

After pacing, a start and end point of a bipolar window may be determined as schematically indicated in FIG. 8. Bipolar windowing refers to a set of signal processing operations for detecting the active part of the bipolar signals, which is performed after a set of operations (far field reduction and pacing removal). Next, each bipolar window is decomposed into Hermite basis functions and the resulting coefficients and width parameter are used to represent the bipolar window. This method has been applied successfully for clustering QRS complexes. Each bipolar window is centered at the location of its maximum peaks and modeled as a combination of Hermite basis: $x \left(t\right) = {\sum }_{n = 0}^{N} {c}_{n} \left(σ\right) ⋅ {H}_{n} \left(t, σ\right) + e \left(t, σ\right)$ (1) where *H* - denote Hermitian polynomials. *N* - order of Hermite basis it is a configurable number between 12-24, *σ* - width of the basis and *cₙ* (σ) *=* Σ*ₜx*(*t*) · *Hₙ*(*t*, *σ*); are the coefficients. The parameters *cₙ*(σ)*,* and *σ* are selected to minimize the error: ${\sum }_{t} {\left|e\left(t, σ\right)\right|}^{2} = {{\sum }_{t} \left|x\left(t\right)-{\sum }_{n = 0}^{N} {c}_{n} \left(σ\right) ⋅ {H}_{n} \left(t, σ\right)\right|}^{2}$ (2)

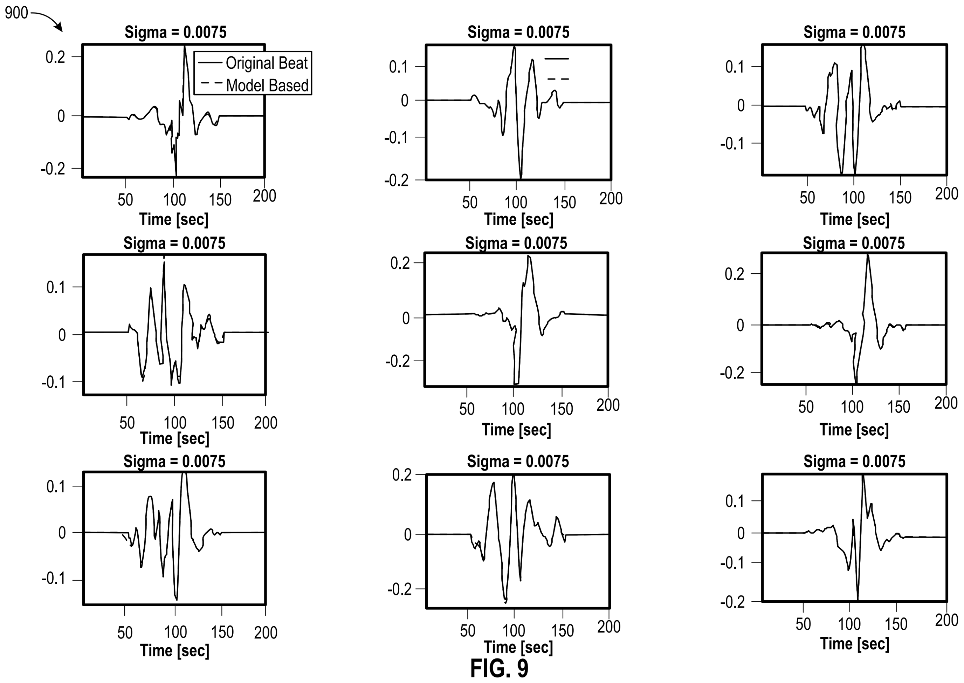

Accordingly, an example of modeling different waves with Hermite basis function (N =8) for reconstruction of a bipolar window of different waves with a set of coefficients is depicted in FIG. 9.

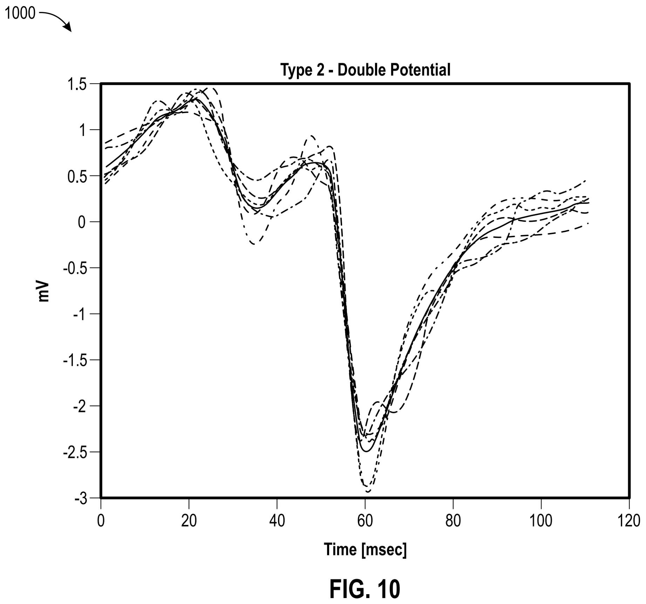

Next, an unsupervised clustering of the beats may be performed based on the similarities between the coefficients, presenting a "family" of beats." A physician annotates for each family the type of beats (normal, double potential, fractionation or noise), such as depicted in FIG. 10. As will be appreciated, this reduces the need to review annotations beat by beat. Subsequently, a random forest decision tree may be performed to take a set of Hermitian coefficients and the annotated labels in order to label each beat as one of the possible beat categories.

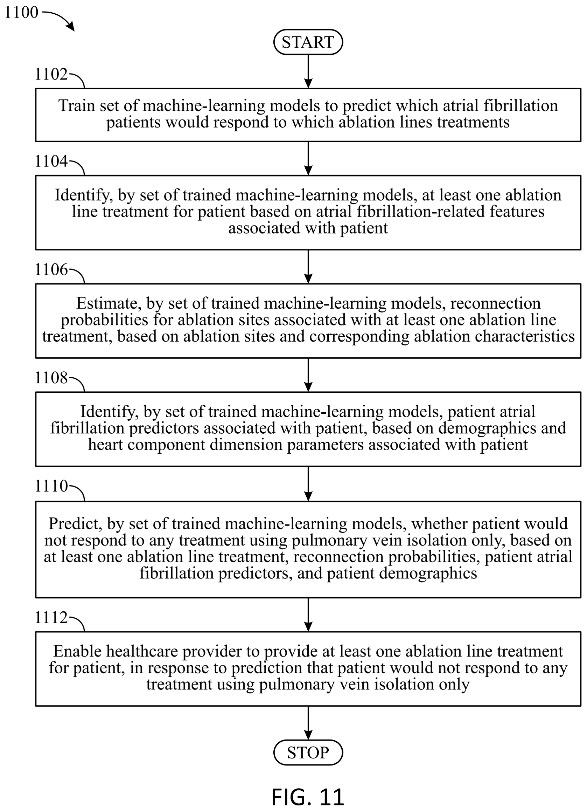

FIG. 11 is a flowchart that illustrates a computer-implemented method for using deep-learning for identifying pulmonary vein isolation non-responders, under an embodiment. Flowchart **1100** depicts method acts illustrated as flowchart blocks for certain actions involved in and/or between the system elements **102 - 144** of FIG. 1.

A set of machine-learning models is trained to predict which atrial fibrillation patients would respond to which ablation lines treatments, block **1102.** The system trains neural networks to differentiate between ablation line treatments to which each patient can respond. For example, and without limitation, this may include a training system training a set of machine-learning models to predict which atrial fibrillation patients would respond to which ablation lines, treatments, such as one patient would respond to a pulmonary vein isolation only treatment while another patient would respond to a pulmonary vein isolation plus treatment.

A machine-learning model can be an application of artificial intelligence that provides a system with the ability to automatically improve from experience without being explicitly programmed. An atrial fibrillation patient can be a person receiving or registered to receive medical treatment for the most commonly diagnosed sustained arrhythmia, which is characterized by rapid and irregular activation of the upper cavities of the heart. A treatment can be medical care given to a patient for an illness or injury. An ablation line can be a long, narrow mark or band where a lesion is surgically created that may interrupt the abnormal electrical pathways causing arrhythmias. An ablation line treatment can be medical care that surgically creates a lesion at a long, narrow mark or band, whicht may interrupt the patient's abnormal electrical pathways causing arrhythmias.

After the training, the set of trained machine-learning models identifies at least one ablation line treatment associated with a patient, based on atrial fibrillation-related features associated with the patient, block **1104.** The system uses medical imaging to identify a patient's ablation lines. By way of example and without limitation, this may include the set of trained machine-learning models **100** extracting maps from medical images such as specific intracardiac electrocardiogram data, for a 61-year old patient named Pat, and correlating the mapping data with a treatment that uses 33 ablation sites in 6 ablation lines.

A trained machine-learning model can be an application of artificial intelligence that has been developed to enable a system to automatically improve from experience without being explicitly programmed. An ablation site can be the area or location where a lesion is surgically created that may interrupt the abnormal electrical pathways causing arrhythmias. A patient can be a person receiving or registered to receive medical treatment. An atrial fibrillation-related feature can be a distinctive attribute associated with an irregular and often rapid heart rhythm characterized by the heart beating chaotically and irregularly..

Having identified at least one ablation line treatment, the set of trained machine-learning models estimates reconnection probabilities for ablation sites associated with the at least one ablation line treatment, based on the ablation sites and the corresponding ablation characteristics, block **1106.** The system determines which sites that have been ablated may spontaneously reconnect. For example, and without limitation, this may include the set of trained machine-learning models **100** extracting mapping data from the data for the 33 ablation sites and the corresponding ablation characteristics, which include an impedance drop, a radiofrequency index, catheter stability, catheter power, and/or catheter force, and then storing the mapping data in an accessible data structure.,

A reconnection probability can be an estimated recurrence of atrial fibrillation after catheter ablation. A corresponding ablation characteristic can be a relationship to a feature or quality typically associated with the surgical creation of a lesion that may interrupt the abnormal electrical pathways causing arrhythmias.

In addition to suggesting ablation lines and reconnection sites, the set of trained machine-learning models identifies atrial fibrillation predictors associated with the patient, based on demographics and heart component dimension parameters associated with the patient, block **1108.** The system identifies the predictors for the patient's atrial fibrillation. By way of example and without limitation, this may include the set of trained machine-learning models **100,** receiving the patient's demographics data and right atrial parameters, which include atrium size, atrium sphericity, low voltage zones, global voltage, and/or cycle length, and which the set of trained machine-learning models uses to identify Pat's atrial fibrillation predictors.

A patient atrial fibrillation predictor can be forecasters of the most commonly diagnosed sustained arrhythmia, which is characterized by rapid and irregular activation of the heart. A demographic can be the statistical characteristics of human populations. A heart dimension parameter can be a numerical or other measurable factor that defines a cardiac component.

After identifying the at least one ablation line treatment, the reconnection probabilities, and the arial fibrillation predictors, the set of trained machine-learning models predicts whether the patient would respond to a treatment using pulmonary vein isolation only, which is based on the at least one ablation line treatment, the estimated reconnection probabilities for the ablation sites, and any patient atrial fibrillation predictors, block **1110.** The system predicts whether a patient's atrial fibrillation is likely to respond to a pulmonary vein isolation only treatment. In embodiments, this may include the set of trained machine-learning models **100,** receiving data which includes the 6 ablation lines treatment that include the 33 ablation sites, the highest estimated reconnection probabilities for ablation sites, and Pat's atrial fibrillation predictors, to predict that Pat's atrial fibrillation condition would not respond to treatment using the pulmonary vein isolation only treatment. Then the system **100** can output this prediction **144** to Pat's physician.

A prediction can be a forecast, what someone thinks will happen, or a statement about a future event or about future data. Pulmonary vein isolation only can be a medical procedure that aims to restore normal heart rhythm in the left atrium and reduce or eliminate the symptoms associated with atrial fibrillation.

In response to a prediction that a patient would not respond to any treatment using pulmonary vein isolation only, a healthcare provider is enabled to provide the at least one ablation line treatment for the patient, block **1112.** The system enables a pulmonary vein isolation plus treatment for patients who need more than a pulmonary vein isolation only treatment. By way of example and without limitation, this may include the set of trained machine-learning models **100** outputting the detailed coordinates of the 33 ablation sites in the 6 ablation lines in Pat's right atrium which were identified for Pat the patient, thereby enabling Pat's physician to provide a procedure that otherwise would not have been provided. A healthcare provider can be an individual or organization that offers medical services. The at least one ablation line treatment is a line of ablation sites which is organized as an ablation line one of when identified for the patient or when enabled for the healthcare provider. This means that the ablation line treatment can be formed as a line when output as the ablation line prediction **114,** or after being processed as ablation sites by the ARMA softmax and subsequently formed into a line by the final dense layer **130.**

In addition to using one of the trained machine-learning models to predict whether a patient is a responder to a treatment using pulmonary vein isolation, the trained machine-learning models can predict which ablation approach is most likely to yield the better outcome for the patient. In other words, the trained machine-learning models may be used to recommend an ablation approach that includes pulmonary vein isolation treatment only (typically involving WACA ablation), or "pulmonary vein isolation plus" treatment, which includes ablation strategies beyond WACA ablation. These alternative ablation strategies may include, but are not limited to, 1) Anatomical Ablation to isolate or cross anatomical segments/structures/locations in the atrium, 2) Linear Ablation according to EA map findings regardless of map type, which can be composed of any combination of ablation lines, including but not limited to: roof line, inferior line, mitral line (different types), anterior line, LAA isolation, SVC isolation etc., 3) Focal Ablation, including but not limited to, Fractionation ablation, CARTOFINDER ablation, Fast CL ablation, Low voltage ablation and/or homogenization, Isuprel mapping and ablation / breakthrough ablation, slow conduction corridors and pivot points ablation, GP ablation, Spatial Temporal Dispersion ablation and others, and 4) Focal Anatomical Ablation, which may include debulking of specific segments/structures through focal ablations. Therefore, in the event the model predicts that a patient's atrial fibrillation condition would not respond to treatment using pulmonary vein isolation only, the model can recommend an ablation approach for that patient that would lead to a successful long-term outcome, i.e., pulmonary vein isolation only or pulmonary vein isolation plus, including the type of pulmonary vein isolation plus that would most likely yield a better outcome for the patient.

Although FIG. 11 depicts the blocks **1102 - 1112** occurring in a specific order, the blocks **1102 - 1112** may occur in another order. In other implementations, each of the blocks **1102 - 1112** may also be executed in combination with other blocks and/or some blocks may be divided into a different set of blocks.

### System Overview

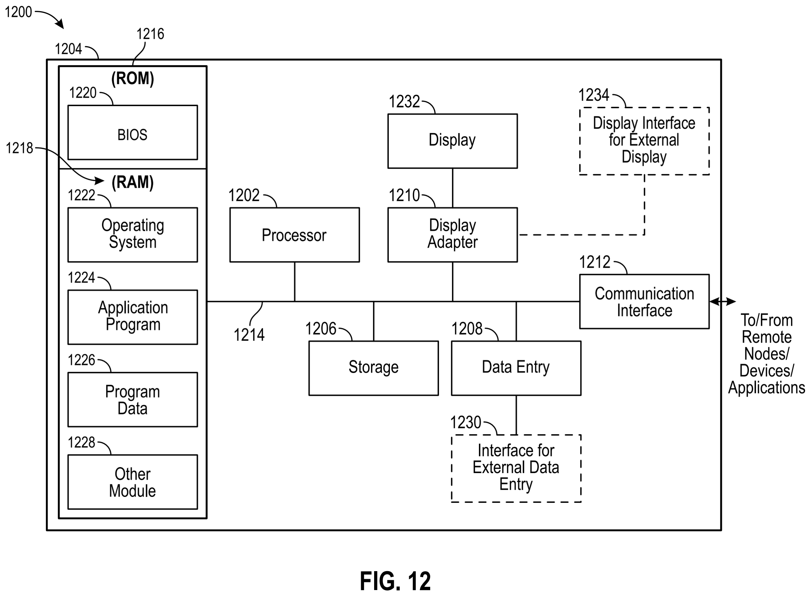

In an exemplary hardware device in which the subject matter may be implemented shall be described. Those of ordinary skill in the art will appreciate that the elements illustrated in FIG. 12 may vary depending on the system implementation. With reference to FIG. 12, an exemplary system for implementing the subject matter disclosed herein includes a hardware device **1200,** including a processing unit **1202,** a memory **1204,** a storage **1206,** a data entry module **1208,** a display adapter **1210,** a communication interface **1212,** and a bus **1214** that couples elements **1204 - 1212** to the processing unit **1202.**

The bus **1214** may comprise any type of bus architecture. Examples include a memory bus, a peripheral bus, a local bus, etc. The processing unit **1202** is an instruction execution machine, apparatus, or device and may comprise a microprocessor, a digital signal processor, a graphics processing unit, an application specific integrated circuit (ASIC), a field programmable gate array (FPGA), etc. The processing unit **1202** may be configured to execute program instructions stored in the memory **1204** and/or the storage **1206** and/or received via the data entry module **1208.**

The memory **1204** may include a read only memory (ROM) **1216** and a random-access memory (RAM) **1218.** The memory **1204** may be configured to store program instructions and data during operation of the hardware device **1200.** In various embodiments, the memory **1204** may include any of a variety of memory technologies such as static random-access memory (SRAM) or dynamic RAM (DRAM), including variants such as dual data rate synchronous DRAM (DDR SDRAM), error correcting code synchronous DRAM (ECC SDRAM), or RAMBUS DRAM (RDRAM), for example. The memory **1204** may also include nonvolatile memory technologies such as nonvolatile flash RAM (NVRAM) or ROM. In some embodiments, it is contemplated that the memory **1204** may include a combination of technologies such as the foregoing, as well as other technologies not specifically mentioned. When the subject matter is implemented in a computer system, a basic input/output system (BIOS) **1220,** containing the basic routines that help to transfer information between elements within the computer system, such as during start-up, is stored in the ROM **1216.**

The storage **1206** may include a flash memory data storage device for reading from and writing to flash memory, a hard disk drive for reading from and writing to a hard disk, a magnetic disk drive for reading from or writing to a removable magnetic disk, and/or an optical disk drive for reading from or writing to a removable optical disk such as a CD ROM, DVD or other optical media. The drives and their associated computer-readable media provide nonvolatile storage of computer readable instructions, data structures, program modules and other data for the hardware device **1200.**

It is noted that the methods described herein may be embodied in executable instructions stored in a computer readable medium for use by or in connection with an instruction execution machine, apparatus, or device, such as a computer-based or processor-containing machine, apparatus, or device. It will be appreciated by those skilled in the art that for some embodiments, other types of computer readable media may be used which may store data that is accessible by a computer, such as magnetic cassettes, flash memory cards, digital video disks, Bernoulli cartridges, RAM, ROM, and the like may also be used in the exemplary operating environment. As used here, a "computer-readable medium" may include one or more of any suitable media for storing the executable instructions of a computer program in one or more of an electronic, magnetic, optical, and electromagnetic format, such that the instruction execution machine, system, apparatus, or device may read (or fetch) the instructions from the computer readable medium and execute the instructions for carrying out the described methods. A non-exhaustive list of conventional exemplary computer readable medium includes: a portable computer diskette; a RAM; a ROM; an erasable programmable read only memory (EPROM or flash memory); optical storage devices, including a portable compact disc (CD), a portable digital video disc (DVD), a high-definition DVD (HD-DVD^{™}), a BLU-RAY disc; and the like.

A number of program modules may be stored on the storage **1206,** the ROM **1216** or the RAM **1218,** including an operating system **1222,** one or more applications programs **1224,** program data **1226,** and other program modules **1228.** A user may enter commands and information into the hardware device **1200** through data entry module **1208.** The data entry module **1208** may include mechanisms such as a keyboard, a touch screen, a pointing device, etc.

Other external input devices (not shown) are connected to the hardware device **1200** via an external data entry interface **1230.** By way of example and not limitation, external input devices may include a microphone, joystick, game pad, satellite dish, scanner, or the like. In some embodiments, external input devices may include video or audio input devices such as a video camera, a still camera, etc. The data entry module **1208** may be configured to receive input from one or more users of the hardware device **1200** and to deliver such input to the processing unit **1202** and/or the memory **1204** via the bus **1214.**

A display **1232** is also connected to the bus **1214** via the display adapter **1210.** The display **1232** may be configured to display output of the hardware device **1200** to one or more users. In some embodiments, a given device such as a touch screen, for example, may function as both the data entry module **1208** and the display **1232.** External display devices may also be connected to the bus **1214** via the external display interface **1234.** Other peripheral output devices, not shown, such as speakers and printers, may be connected to the hardware device **1200.**

The hardware device **1200** may operate in a networked environment using logical connections to one or more remote nodes (not shown) via the communication interface **1212.** The remote node may be another computer, a server, a router, a peer device or other common network node, and typically includes many or all of the elements described above relative to the hardware device **1200.** The communication interface **1212** may interface with a wireless network and/or a wired network. Examples of wireless networks include, for example, a BLUETOOTH network, a wireless personal area network, a wireless 802.11 local area network (LAN), and/or wireless telephony network (e.g., a cellular, PCS, or GSM network). Examples of wired networks include, for example, a LAN, a fiber optic network, a wired personal area network, a telephony network, and/or a wide area network (WAN). Such networking environments are commonplace in intranets, the Internet, offices, enterprise-wide computer networks and the like. In some embodiments, the communication interface **1212** may include logic configured to support direct memory access (DMA) transfers between the memory **1204** and other devices.

In a networked environment, program modules depicted relative to the hardware device **1200,** or portions thereof, may be stored in a remote storage device, such as, for example, on a server. It will be appreciated that other hardware and/or software to establish a communications link between the hardware device **1200** and other devices may be used.

It should be understood that the arrangement of the hardware device **1200** illustrated in FIG. **12** is but one possible implementation on and that other arrangements are possible. It should also be understood that the various system components (and means) defined by the claims, described below, and illustrated in the various block diagrams represent logical components that are configured to perform the functionality described herein. For example, one or more of these system components (and means) may be realized, in whole or in part, by at least some of the components illustrated in the arrangement of the hardware device **1200.**

In addition, while at least one of these components are implemented at least partially as an electronic hardware component, and therefore constitutes a machine, the other components may be implemented in software, hardware, or a combination of software and hardware. More particularly, at least one component defined by the claims is implemented at least partially as an electronic hardware component, such as an instruction execution machine (e.g., a processor-based or processor-containing machine) and/or as specialized circuits or circuitry (e.g., discrete logic gates interconnected to perform a specialized function), such as those illustrated in FIG. **12****.**

Other components may be implemented in software, hardware, or a combination of software and hardware. Moreover, some or all of these other components may be combined, some may be omitted altogether, and additional components may be added while still achieving the functionality described herein. Thus, the subject matter described herein may be embodied in many different variations, and all such variations are contemplated to be within the scope of what is claimed.

Consistent with the foregoing, in at least one case referred to herein as Example A-1, a system for using deep-learning for identifying pulmonary vein isolation only which ablation line treatments comprises one or more processors and a non-transitory computer readable medium storing a plurality of instructions, which when executed, cause the one or more processors to: train a set of machine-learning models to predict which atrial fibrillation patients would respond to which treatments using ablation lines; identify, by the set of trained machine-learning models, at least one ablation line treatment for a patient based on atrial fibrillation-related features associated with the patient; estimate, by the set of trained machine-learning models, reconnection probabilities for ablation sites associated with the at least one ablation line treatment, based on the ablation sites and corresponding ablation characteristics; identify, by the set of trained machine-learning models, patient atrial fibrillation predictors associated with the patient, based on demographics and heart component dimension parameters associated with the patient; predict, by the set of trained machine-learning models, whether the patient would not respond to any treatment using pulmonary vein isolation only, based on the at least one ablation line treatment, the reconnection probabilities, the patient atrial fibrillation predictors, and the patient demographics; and enable a healthcare provider to provide the at least one ablation line treatment for the patient, in response to a prediction that the patient would not respond to any treatment using pulmonary vein isolation only.

In another case referred to herein as Example A-2, the system of Example A-1, or any other exemplary embodiment described herein, may be further limited wherein: training the set of machine-learning models comprises using a training data set which includes a number of records of atrial fibrillation patients which record observed outcomes from atrial fibrillation treatments, and the number of records exceeds a training records threshold for observed outcomes.

In another case referred to herein as Example A-3, the system of Example A-1, or any other exemplary embodiment described herein, may be further limited wherein: in response to the number of records failing to exceed the training records threshold for observed outcomes, training the set of machine-learning models comprises using a training data set which includes a number of records of atrial fibrillation patients which record healthcare provider ablation approaches for atrial fibrillation treatments, and the number of records exceeds a training records threshold for ablation approaches recommended by healthcare providers, and wherein identifying the at least one ablation line treatment for the patient is based on the ablation approaches recommended by healthcare provider.

In another case referred to herein as Example A-4, the system of Example A-1, or any other exemplary embodiment described herein, may be further limited wherein: predicting whether the patient would not respond to any treatment using pulmonary vein isolation only is based on training the set of machine-learning models using a training data set which includes only records of atrial fibrillation patients which record treatment using pulmonary vein isolation only.

In another case referred to herein as Example A-5, the system of Example A-1, or any other exemplary embodiment described herein, may be further limited wherein: identifying the at least one ablation line for the patient includes predicting a probability of success for the treatment regardless of ablation approach.

In another case referred to herein as Example A-6, the system of Example A-1, or any other exemplary embodiment described herein, may be further limited wherein: the at least one ablation line treatment is directed to a location other than the left atrium, and is therefore a pulmonary vein isolation plus treatment rather than a pulmonary vein isolation only treatment.

In another case referred to herein as Example A-7, the system of Example A-1, or any other exemplary embodiment described herein, may be further limited wherein: the at least one ablation line treatment comprises a line of ablation sites which is organized as an ablation line one of when identified for the patient or when enabled for the healthcare provider.

Consistent with the foregoing, in at least one case referred to herein as Example B-1, a method for using deep-learning for identifying pulmonary vein isolation only non-responders comprises: training a set of machine-learning models to predict which atrial fibrillation patients would respond to which treatments using ablation lines; identifying, by the set of trained machine-learning models, at least one ablation line treatment for a patient based on atrial fibrillation-related features associated with the patient; estimating, by the set of trained machine-learning models, reconnection probabilities for ablation sites associated with the at least one ablation line treatment, based on the ablation sites and corresponding ablation characteristics; identifying, by the set of trained machine-learning models, patient atrial fibrillation predictors associated with the patient, based on demographics and heart component dimension parameters associated with the patient; predicting by the set of trained machine-learning models, whether the patient would not respond to any treatment using pulmonary vein isolation only, based on the at least one ablation line treatment, the reconnection probabilities, the patient atrial fibrillation predictors, and the patient demographics; and enabling a healthcare provider to provide the at least one ablation line treatment for the patient, in response to a prediction that the patient would not respond to any treatment using pulmonary vein isolation only.

In another case referred to herein as Example B-2, the method of Example B-1, or any other exemplary embodiment described herein, may be further limited wherein: training the set of machine-learning models comprises using a training data set which includes a number of records of atrial fibrillation patients which record observed outcomes from atrial fibrillation treatments, and the number of records exceeds a training records threshold for observed outcomes.

In another case referred to herein as Example B-3, the method of Example B-1, or any other exemplary embodiment described herein, may be further limited wherein: in response to the number of records failing to exceed the training records threshold for observed outcomes, training the set of machine-learning models comprises using a training data set which includes a number of records of atrial fibrillation patients which record healthcare provider ablation approaches for atrial fibrillation treatments, and the number of records exceeds a training records threshold for ablation approaches recommended by healthcare providers, and wherein identifying the at least one ablation line treatment for the patient is based on the ablation approaches recommended by healthcare providers.

In another case referred to herein as Example B-4, the method of Example B-1, or any other exemplary embodiment described herein, may be further limited wherein: predicting whether the patient would not respond to any treatment using pulmonary vein isolation only is based on training the set of machine-learning models using a training data set which includes only records of atrial fibrillation patients which record treatment using pulmonary vein isolation only.

In another case referred to herein as Example B-5, the method of Example B-1, or any other exemplary embodiment described herein, may be further limited wherein: identifying the at least one ablation line for the patient includes predicting a probability of success for the treatment regardless of ablation approach.

In another case referred to herein as Example B-6, the method of Example B-1, or any other exemplary embodiment described herein, may be further limited wherein: the at least one ablation line treatment is directed to a location other than the left atrium, and is therefore a pulmonary vein isolation plus treatment rather than a pulmonary vein isolation only treatment.

In another case referred to herein as Example B-7, the method of Example B-1, or any other exemplary embodiment described herein, may be further limited wherein: the at least one ablation line treatment comprises a line of ablation sites which is organized as an ablation line one of when identified for the patient or when enabled for the healthcare provider

Consistent with the foregoing, in at least one case referred to herein as Example C-1, a computer program product, comprising a non-transitory computer-readable medium having a computer-readable program code embodied therein to be executed by one or more processors, comprises program code that includes instructions to: train a set of machine-learning models to predict which atrial fibrillation patients would respond to which treatments using ablation lines; identify, by the set of trained machine-learning models, at least one ablation line treatment for a patient based on atrial fibrillation-related features associated with the patient; estimate, by the set of trained machine-learning models, reconnection probabilities for ablation sites associated with the at least one ablation line treatment, based on the ablation sites and corresponding ablation characteristics; identify, by the set of trained machine-learning models, patient atrial fibrillation predictors associated with the patient, based on demographics and heart component dimension parameters associated with the patient; predict, by the set of trained machine-learning models, whether the patient would not respond to any treatment using pulmonary vein isolation only, based on the at least one ablation line treatment, the reconnection probabilities, the patient atrial fibrillation predictors, and the patient demographics; and enable a healthcare provider to provide the at least one ablation line treatment for the patient, in response to a prediction that the patient would not respond to any treatment using pulmonary vein isolation only.

In another case referred to herein as Example C-2, the computer program product of Example C-1, or any other exemplary embodiment described herein, may be further limited wherein: training the set of machine-learning models comprises using a training data set which includes a number of records of atrial fibrillation patients which record observed outcomes from atrial fibrillation treatments, and the number of records exceeds a training records threshold for observed outcomes.

In another case referred to herein as Example C-3, the computer program product of Example C-1, or any other exemplary embodiment described herein, may be further limited wherein: in response to the number of records failing to exceed the training records threshold for observed outcomes, training the set of machine-learning models comprises using a training data set which includes a number of records of atrial fibrillation patients which record healthcare provider ablation approaches for atrial fibrillation treatments, and the number of records exceeds a training records threshold for ablation approaches recommended by healthcare providers, and wherein identifying the at least one ablation line treatment for the patient is based on the ablation approaches recommended by healthcare providers.

In another case referred to herein as Example C-4, the computer program product of Example C-1, or any other exemplary embodiment described herein, may be further limited wherein: predicting whether the patient would not respond to any treatment using pulmonary vein isolation only is based on training the set of machine-learning models using a training data set which includes only records of atrial fibrillation patients which record treatment using pulmonary vein isolation only.

In another case referred to herein as Example C-5, the computer program product of Example C-1, or any other exemplary embodiment described herein, may be further limited wherein: identifying the at least one ablation line for the patient includes predicting a probability of success for the treatment regardless of ablation approach.

In another case referred to herein as Example C-6, the computer program product of Example C-1, or any other exemplary embodiment described herein, may be further limited wherein: the at least one ablation line treatment is directed to a location other than the left atrium, and is therefore a pulmonary vein isolation plus treatment rather than a pulmonary vein isolation only treatment, and wherein the at least one ablation line treatment comprises a line of ablation sites which is organized as an ablation line one of when identified for the patient or when enabled for the healthcare provider.

In the descriptions above, the subject matter is described with reference to acts and symbolic representations of operations that are performed by one or more devices, unless indicated otherwise. As such, it is understood that such acts and operations, which are at times referred to as being computer-executed, include the manipulation by the processing unit of data in a structured form. This manipulation transforms the data or maintains it at locations in the memory system of the computer, which reconfigures or otherwise alters the operation of the device in a manner well understood by those skilled in the art. The data structures where data is maintained are physical locations of the memory that have particular properties defined by the format of the data. However, while the subject matter is described in a context, it is not meant to be limiting as those of skill in the art will appreciate that various of the acts and operations described hereinafter may also be implemented in hardware.

To facilitate an understanding of the subject matter described above, many aspects are described in terms of sequences of actions. At least one of these aspects defined by the claims is performed by an electronic hardware component. For example, it will be recognized that the various actions may be performed by specialized circuits or circuitry, by program instructions being executed by one or more processors, or by a combination of both. The description herein of any sequence of actions is not intended to imply that the specific order described for performing that sequence must be followed. All methods described herein may be performed in any suitable order unless otherwise indicated herein or otherwise clearly contradicted by context.

While one or more implementations have been described by way of example and in terms of the specific embodiments, it is to be understood that one or more implementations are not limited to the disclosed embodiments. To the contrary, it is intended to cover various modifications and similar arrangements as would be apparent to those skilled in the art. Therefore, the scope of the appended claims should be accorded the broadest interpretation so as to encompass all such modifications and similar arrangements.

## Claims

1. A system for using deep-learning for identifying pulmonary vein isolation non-responders, the system comprising:
one or more processors; and
a non-transitory computer readable medium storing a plurality of instructions, which when executed, cause the one or more processors to:
train a set of machine-learning models to predict which atrial fibrillation patients would respond to which treatments using ablation lines;
identify, by the set of trained machine-learning models, at least one ablation line treatment for a patient based on atrial fibrillation-related features associated with the patient;
estimate, by the set of trained machine-learning models, reconnection probabilities for ablation sites associated with the at least one ablation line treatment, based on the ablation sites and corresponding ablation characteristics;
identify, by the set of trained machine-learning models, patient atrial fibrillation predictors associated with the patient, based on demographics and heart component dimension parameters associated with the patient;
predict, by the set of trained machine-learning models, whether the patient would not respond to any treatment using pulmonary vein isolation only, based on the at least one ablation line treatment, the reconnection probabilities, the patient atrial fibrillation predictors, and the patient demographics; and
enable a healthcare provider to provide the at least one ablation line treatment for the patient, in response to a prediction that the patient would not respond to any treatment using pulmonary vein isolation only.

2. The system of claim 1, wherein training the set of machine-learning models comprises using a training data set which includes a number of records of atrial fibrillation patients which record observed outcomes from atrial fibrillation treatments, and the number of records exceeds a training records threshold for observed outcomes, optionally wherein in response to the number of records failing to exceed the training records threshold for observed outcomes, training the set of machine-learning models comprises using a training data set which includes a number of records of atrial fibrillation patients which record healthcare provider ablation approaches for atrial fibrillation treatments, and the number of records exceeds a training records threshold for ablation approaches recommended by healthcare providers, and wherein identifying the at least one ablation line treatment for the patient is based on the ablation approaches recommended by healthcare providers.

3. The system of claim 1, wherein predicting whether the patient would not respond to any treatment using pulmonary vein isolation only is based on training the set of machine-learning models using a training data set which includes only records of atrial fibrillation patients which record treatment using pulmonary vein isolation only.

4. The system of claim 1, wherein identifying the at least one ablation line for the patient includes predicting a probability of success for the treatment regardless of ablation approach.

5. The system of claim 1, wherein the at least one ablation line treatment is directed to a location other than the left atrium, and is therefore a pulmonary vein isolation plus treatment rather than a pulmonary vein isolation only treatment, or the at least one ablation line treatment comprises a line of ablation sites which is organized as an ablation line one of when identified for the patient or when enabled for the healthcare provider.

6. A computer-implemented method for using deep-learning for identifying pulmonary vein isolation non-responders, the computer-implemented method comprising:
training a set of machine-learning models to predict which atrial fibrillation patients would respond to which treatments using ablation lines;
identifying, by the set of trained machine-learning models, at least one ablation line treatment for a patient based on atrial fibrillation-related features associated with the patient;
estimating, by the set of trained machine-learning models, reconnection probabilities for ablation sites associated with the at least one ablation line treatment, based on the ablation sites and corresponding ablation characteristics;
identifying, by the set of trained machine-learning models, patient atrial fibrillation predictors associated with the patient, based on demographics and heart component dimension parameters associated with the patient;
predicting by the set of trained machine-learning models, whether the patient would not respond to any treatment using pulmonary vein isolation only, based on the at least one ablation line treatment, the reconnection probabilities, the patient atrial fibrillation predictors, and the patient demographics; and
enabling a healthcare provider to provide the at least one ablation line treatment for the patient, in response to a prediction that the patient would not respond to any treatment using pulmonary vein isolation only.

7. The computer-implemented method of claim 6, wherein training the set of machine-learning models comprises using a training data set which includes a number of records of atrial fibrillation patients which record observed outcomes from atrial fibrillation treatments, and the number of records exceeds a training records threshold for observed outcomes, optionally wherein in response to the number of records failing to exceed the training records threshold for observed outcomes, training the set of machine-learning models comprises using a training data set which includes a number of records of atrial fibrillation patients which record healthcare provider ablation approaches for atrial fibrillation treatments, and the number of records exceeds a training records threshold for ablation approaches recommended by healthcare providers, and wherein identifying the at least one ablation line treatment for the patient is based on the ablation approaches recommended by healthcare providers.

8. The computer-implemented method of claim 6, wherein predicting whether the patient would not respond to any treatment using pulmonary vein isolation only is based on training the set of machine-learning models using a training data set which includes only records of atrial fibrillation patients which record treatment using pulmonary vein isolation only.

9. The computer-implemented method of claim 6, wherein identifying the at least one ablation line for the patient includes predicting a probability of success for the treatment regardless of ablation approach.

10. The computer-implemented method of claim 6, wherein the at least one ablation line treatment is directed to a location other than the left atrium, and is therefore a pulmonary vein isolation plus treatment rather than a pulmonary vein isolation only treatment, or the at least one ablation line treatment comprises a line of ablation sites which is organized as an ablation line one of when identified for the patient or when enabled for the healthcare provider.

11. A computer program product, comprising a non-transitory computer-readable medium having a computer-readable program code embodied therein to be executed by one or more processors, the program code including instructions to:
train a set of machine-learning models to predict which atrial fibrillation patients would respond to which treatments using ablation lines;
identify, by the set of trained machine-learning models, at least one ablation line treatment for a patient based on atrial fibrillation-related features associated with the patient;
estimate, by the set of trained machine-learning models, reconnection probabilities for ablation sites associated with the at least one ablation line treatment, based on the ablation sites and corresponding ablation characteristics;
identify, by the set of trained machine-learning models, patient atrial fibrillation predictors associated with the patient, based on demographics and heart component dimension parameters associated with the patient;
predict, by the set of trained machine-learning models, whether the patient would not respond to any treatment using pulmonary vein isolation only, based on the at least one ablation line treatment, the reconnection probabilities, the patient atrial fibrillation predictors, and the patient demographics; and
enable a healthcare provider to provide the at least one ablation line treatment for the patient, in response to a prediction that the patient would not respond to any treatment using pulmonary vein isolation only.

12. The computer program product of claim 11, wherein training the set of machine-learning models comprises using a training data set which includes a number of records of atrial fibrillation patients which record observed outcomes from atrial fibrillation treatments, and the number of records exceeds a training records threshold for observed outcomes, optionally wherein in response to the number of records failing to exceed the training records threshold for observed outcomes, training the set of machine-learning models comprises using a training data set which includes a number of records of atrial fibrillation patients which record healthcare provider ablation approaches for atrial fibrillation treatments, and the number of records exceeds a training records threshold for ablation approaches recommended by healthcare providers, and wherein identifying the at least one ablation line treatment for the patient is based on the ablation approaches recommended by healthcare providers.

13. The computer program product of claim 11, wherein predicting whether the patient would not respond to any treatment using pulmonary vein isolation only is based on training the set of machine-learning models using a training data set which includes only records of atrial fibrillation patients which record treatment using pulmonary vein isolation only.

14. The computer program product of claim 11, wherein identifying the at least one ablation line for the patient includes predicting a probability of success for the treatment regardless of ablation approach.

15. The computer program product of claim 11, wherein the at least one ablation line treatment is directed to a location other than the left atrium, and is therefore a pulmonary vein isolation plus treatment rather than a pulmonary vein isolation only treatment, and/or wherein the at least one ablation line treatment comprises a line of ablation sites which is organized as an ablation line one of when identified for the patient or when enabled for the healthcare provider.
